# EUROPEAN PATENT SPECIFICATION

(11) **EP 3 433 846 B1**
(45) Date of publication and mention of the grant of the patent: **09.10.2024**
(21) Application number: 16895716.5
(22) Date of filing: 09.09.2016
(51) Int. Cl.: G09B 23/28, A61M 5/178, A61M 5/32, A61M 5/42, A61M 5/31

(54) **INJECTION TRAINING AND COMPLIANCE DEVICE AND METHOD**
INJEKTIONSTRAININGS- UND -EINHALTUNGSVORRICHTUNG UND -VERFAHREN
DISPOSITIF ET PROCÉDÉ DE FORMATION ET DE CONFORMITÉ D'INJECTION

(30) Priority: 24.03.2016 US 201615079456
(43) Date of publication of application: 30.01.2019
(73) Proprietor: Noble International, Inc., Orlando, FL 32801 (US)
(72) Inventor: BAKER, Jeff, Orlando, FL 32839 (US); SIEMER, Francis, Michael, Orlando, FL 32828 (US); CHUNG, Wai Yin, Christopher, Orlando, FL 32827 (US); BAKER, Craig, Lebanon, OH 45036 (US); FREYTAG, Seth, Winter Park, FL 32792 (US); KOKA, Dinesh, Venkata, Altamonte Springs, FL 32714 (US)
(74) Representative: ABG Intellectual Property Law, S.L.
(86) International application number: PCT/US2016/051030
(87) International publication number: WO 2017/164927

(56) References cited:
- JP-A- 2015 062 563
- US-A- 2 525 398
- US-A- 3 324 854
- US-A1- 2001 044 606
- US-A1- 2001 044 606
- US-A1- 2003 055 436
- US-A1- 2004 147 901
- US-A1- 2010 140 125
- US-A1- 2014 163 526
- US-A1- 2015 025 458
- US-A1- 2015 105 720
- US-B1- 8 527 033

## Description

### BACKGROUND

The use of needles and instruments containing needles are widespread in the medical industry. Needle insertion into a patient is an essential component of many clinical procedures such as biopsies, injections, neurosurgery, and cancer treatment methods. Certain disease states require daily injections, such as diabetes mellitus. The success of these procedures depends on accurate placement and correct technique during insertion of the needle into a target region of a patient. Accuracy of an injection can be hampered by certain factors, including a fear of needles leading to failure to administer an injection, or improper administration of an injection. Lack of knowledge and/or experience in administering injections to oneself or to others may also lead to improper injection administration.

Increased use of biologic and large molecule drugs has driven the demand for prefilled syringes and other drug delivery systems. Prefilled syringes are combination products used in home and institutional settings by patients and health care providers (HCP). Failure to effectively use a prefilled syringe, for example, can result in errors that adversely affect the safe and full delivery of a prescribed dose. Training users of prefilled syringes and other needle-bearing drug delivery systems serves as an attractive strategy to mitigate errors and support the commercial objectives of syringe products and other medication administration products in the market.

Injectable medications are required for a number of varying illnesses and diseases. Many injectable medications require self-injection by a patient. Self-injection of a medicament using a device having a needle carries with it a certain stigma. Oftentimes users are weary of administering an injection for fear or anxiety related to handling an injection device, failing to deliver a complete dose of the medication, anticipated pain associated with injecting the subject with the needle, fear of accidentally sticking themselves with the needle during manipulation of the injection device, and difficulties in adequately grasping the dosing mechanism or injection device to inject the subject, among other concerns. An additional concern exists in instances in which users with little or no medical knowledge or experience are required to inject themselves or another subject using these devices. Performing a medical treatment or test using a device having or requiring a needle carries with it certain risks and often creates a level of anxiety for the user performing the treatment or test and/or for the subject receiving the treatment or test.

Injections are often a source of fear and apprehension for injection providers as well as patients receiving injections. Studies suggest that as many as one-fifth to one-third of people with diabetes, for example, are hesitant or unwilling to give themselves insulin injections for reasons that include needle anxiety ([1], [2], [3]).

Additional concerns arise where particular steps must be followed during an injection, or where the correct positioning of the injection device must be used to prevent harm to the patient and to administer an injection correctly. Un-trained injection providers may not appreciate the deleterious effects that an improperly administered injection may cause including, but not limited to trauma to bone tissue caused by contact with the needle, systemic poisoning as a result of incorrect placement of anesthetic injections and injection into the blood stream, for example, hematoma formation resulting from incorrect placement of the needle followed by aspiration during injections during anesthetic procedures, among other detrimental results of improper technique in positioning and placement of injection devices.

Document US 2001/044606 A1 discloses a needle injection-facilitating device. Document US 2003/055436 A1 discloses a device for the insertion of medical instruments into the human body. Document US 8527033 B1 discloses systems and methods for assisting with internal positioning of instruments. Further examples are disclosed in US2525398A, US2015/025458A1, US2004/147901A1 and US3324854A.

### SUMMARY

A positioning apparatus according to claim 1 and a method according to claim 12 are defined.

In an example, a positioning apparatus for guiding an injection device relative to a target surface having a representative plane is provided. The apparatus includes a housing defining a channel configured to receive an injection device, wherein a first angle is formed between a longitudinal axis of the channel and the representative plane of the target surface so as to guide the injection device through the channel to the target surface at the first angle, and wherein the housing comprises a stabilizing surface configured to abut the target surface to stabilize an injection according to the first angle.

In a further example, a positioning apparatus for guiding an injection device relative to a target surface is provided, wherein the apparatus includes a housing including a body having a generally conical shape, the body comprising a base portion, an apex portion, an outer surface and an inner surface, wherein an aperture is defined at the apex portion. The apparatus further includes a channel disposed adjacent to the inner surface, the channel terminating at the aperture in the apex portion, the channel configured to receive an injection device, wherein a predetermined angle is formed between a longitudinal axis of the channel and a longitudinal axis of the outer surface defined between the base portion and the apex portion, such that when the outer surface of the body is positioned against the target surface, the injection device contacts the target surface through the aperture at the predetermined angle.

In still a further example, a positioning apparatus for guiding an injection device relative to a target surface is provided. The apparatus includes a housing including a first side having an upper edge portion and a lower edge portion, a second side having an upper edge portion and a lower edge portion, and a bottom surface having a first edge portion and a second edge portion. The first edge portion of the bottom surface associates with the lower edge portion of the first side, and the second edge portion of the bottom surface associates with the lower edge portion of the second side defining an opening there between. A visual indicator may be provided on the first and/or second side, wherein the visual indicator may include a marking on the housing, in a non-limiting embodiment, wherein the marking defines an angular placement of an injection device relative to a longitudinal axis of the bottom surface, such that when aligned with the marking, a first angle is formed between a longitudinal axis of the injection device and the longitudinal axis of the bottom surface of the housing.

In yet a further example, a housing having a support surface for guiding an injection device is provided, wherein the housing is configured to facilitate control of the vector of the injection device relative to a target surface having a representative plane at a first angle relative to the representative plane. The housing includes a stabilizing surface configured to abut the target surface to stabilize an injection at the target surface according to the first angle.

In still a further example, an injection device including a housing having a support surface for guiding an injection device to a target point on a target surface having a representative plane, wherein the housing is configured to facilitate control of the vector of the injection device relative to a target surface at a first angle, the housing comprising a stabilizing surface configured to abut the target surface to stabilize an injection at the target surface according to the first angle.

In another example, a method for positioning an injection device at a target point of a target surface along a first angle relative to the target surface, the target surface having a representative plane is provided. The method includes positioning a positioning apparatus comprising a housing having a guide and being configured to receive an injection device, wherein the guide forms a first angle between a longitudinal axis of the guide and the representative plane of the target surface so as to guide an injection device to the target surface at the first angle and wherein said housing includes a stabilizing surface to stabilize an injection at the target surface according to the first angle, such that the stabilizing surface of the housing abuts the target surface, and a distal portion of the guide is in alignment with the target point of the target surface, placing the injection device onto the housing in alignment with the guide at the first angle, and gliding the injection device to the target point.

### BRIEF DESCRIPTION OF THE DRAWINGS

A more particular description briefly stated above will be rendered by reference to specific embodiments thereof that are illustrated in the appended drawings. Understanding that these drawings depict only typical embodiments and are not therefore to be considered to be limiting of its scope, the embodiments will be described and explained with additional specificity and detail through the use of the accompanying drawings in which:
FIGS. 1A-1B include side views of an embodiment of a positioning apparatus for guiding an injection device relative to a target surface.
FIGS 1C-1D are perspective views of the embodiment shown in FIGS. 1A-1B.
FIGS. 2A-2B include side views of another embodiment of a positioning apparatus for guiding an injection device relative to a target surface.
FIGS. 3A-3B include side views of a further embodiment of a positioning apparatus for guiding an injection device relative to a target surface.
FIG. 3C is a perspective view of the embodiment of the positioning apparatus shown in FIGS. 3A-3B.
FIGS. 4A-4D include side views of still a further embodiment of a positioning apparatus for guiding an injection device relative to a target surface.
FIGS. 5A-5B include a side view of another embodiment of a positioning apparatus for guiding an injection device relative to a target surface.
FIGS. 5C-5D are perspective views of the embodiment of the positioning apparatus shown in FIGS. 5A-5B.
FIGS. 6A-6B include side views of an embodiment of a positioning apparatus for guiding an injection device relative to a target surface having a visual indicator.
FIG. 7 includes a side view of an embodiment of a positioning apparatus comprising a sensor in wireless communication with an external device.
FIGS. 8A-8B include side views of an embodiment of a positioning apparatus associated with an attachment component.
FIGS. 9A-9C include side views of an embodiment of an adjustable positioning apparatus.
FIG. 10 includes a perspective view of an embodiment of a positioning apparatus having a platform extending from a stabilizing surface.
FIG. 11 provides an illustration of an example of a target surface having a target point, showing a non-limiting example of a representative plane on the target surface.
FIG. 12 provides a bottom view of a non-limiting embodiment of a positioning apparatus providing at least two points (a, b,) which contact the target surface.
FIG. 13A is a side view of a further embodiment of a positioning apparatus.
FIG. 13B is a front view of the embodiment shown in FIG. 13A.
FIG. 13C is a perspective view of the embodiment shown in FIGS. 13A-13B.
FIGS. 13D-13E show perspective, side and front views of the positioning apparatus shown in FIGS. 13A-13C in use.
FIGS 14A-14C provide side, front and perspective views, respectively, of a further embodiment of a positioning apparatus in accordance with the invention.
FIGS. 14D-14F provide perspective, side and front views, respectively, of the embodiment of the positioning apparatus in FIGS. 14A-14C in use.

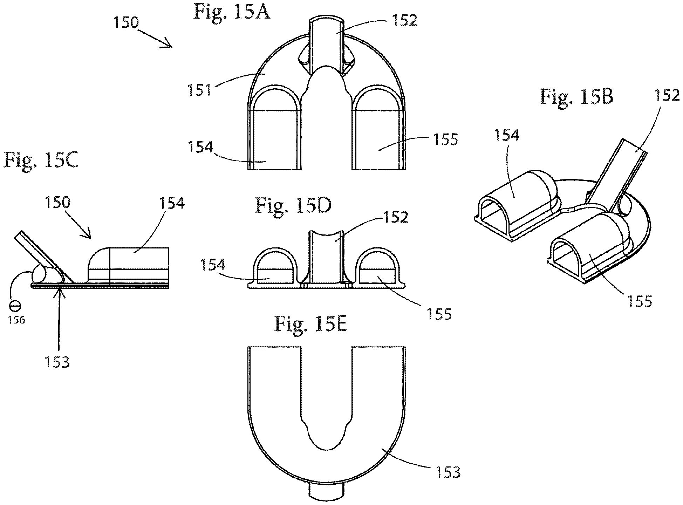

FIG. 15A is a top view of a further embodiment of a positioning apparatus.
FIG. 15B is a perspective view of the positioning apparatus embodiment shown in FIG. 15A.
FIG. 15C is a side view of the positioning apparatus embodiment shown in FIG. 15A.
FIG. 15D is a front view of the positioning apparatus embodiment shown in FIG. 15A.
FIG. 15E is a bottom view of the positioning apparatus embodiment shown in FIG. 15A.
FIG. 16A-E shows different views of an injection assistive apparatus embodiment. FIG. 16A is a front view. FIG. 16B is a cross-sectional view along the A-A axis shown in FIG. 16A. FIG. 16C shows a side front perspective view with base in open state. FIG. 16D shows a side perspective view with base in closed state. FIG. 16E shows a side back perspective view.
FIG. 17A-B shows an injection assistive apparatus. FIG. 17A shows a side front perspective view with a base in an opened state. FIG. 17B is a side-front perspective view with the base in a closed state.

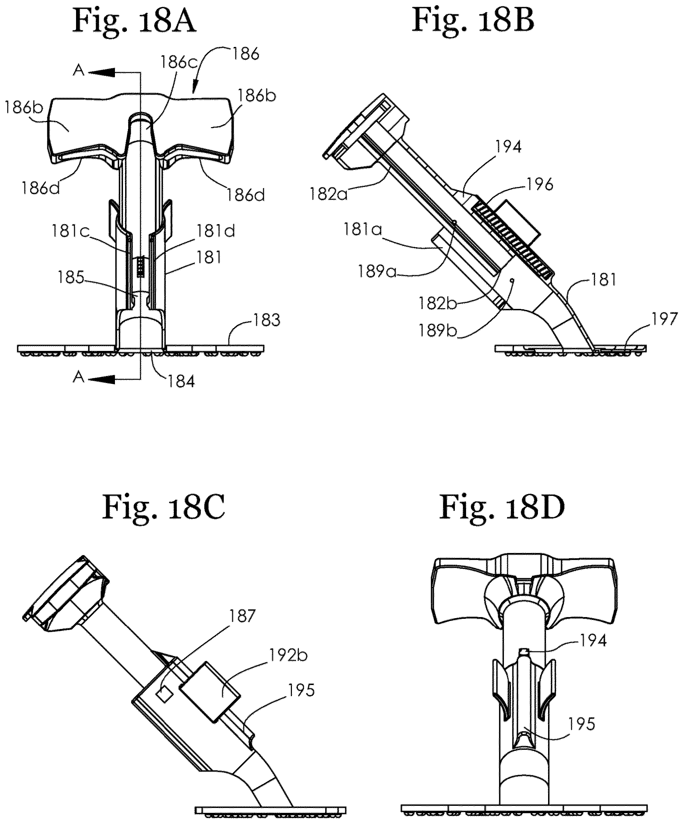

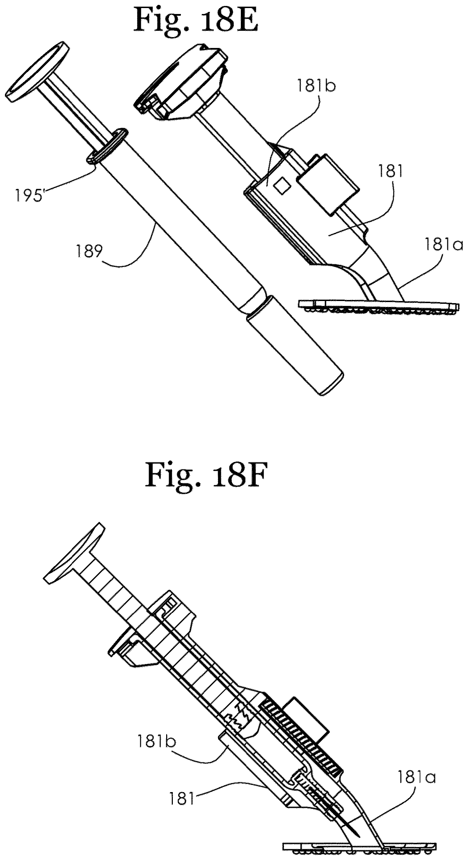

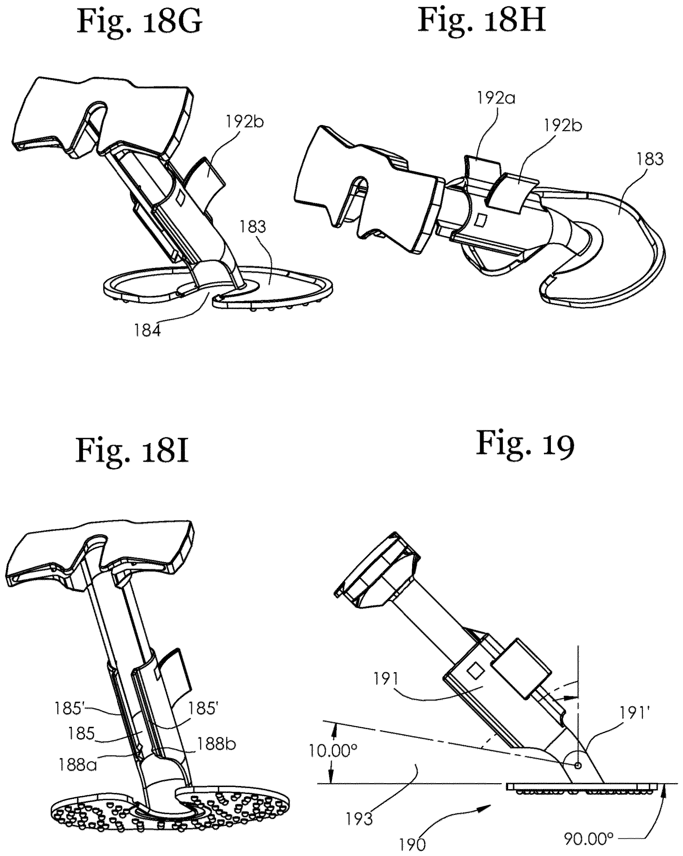

FIG. 18A-I shows different views of an injection assistive apparatus embodiment. FIG. 18A shows a back perspective. FIG. 18B shows a cross-sectional view along the B-B axis shown in FIG. 18A. FIG. 18C shows a side perspective. FIG. 18D shows a front perspective. FIG. 18E shows a side perspective and also an injection device for insertion into the injection assistive apparatus embodiment. FIG. 18F shows a cross-sectional view along the B-B axis of FIG. 18A with the injection device loaded into the apparatus. FIG. 18G shows a side back perspective of the apparatus. FIG. 18H shows a top perspective view of the apparatus. FIG. 18I shows a bottom perspective view of the apparatus.
FIG. 19 shows an injection assistive apparatus similar to that shown in FIG. 18A-I with a pivotable housing.
FIGS. 20A-B show perspective front-side views of an embodiment of an injection assistive apparatus with an injection device loaded into the apparatus.
FIGS. 21A-B show perspective rear-side views of the embodiment of the injection assistive apparatus shown in FIGS. 20A-B with an injection device loaded into the apparatus.

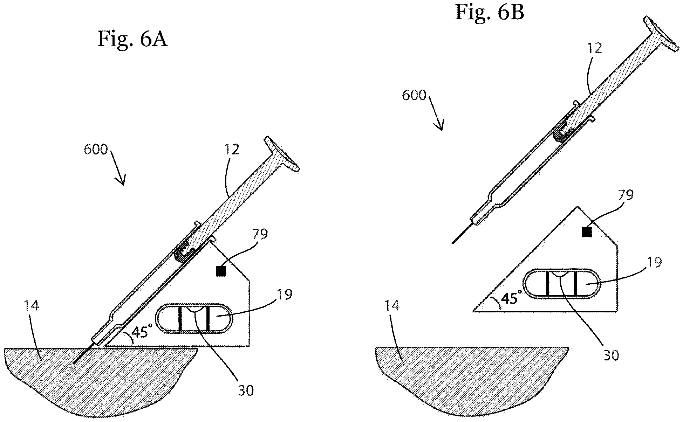

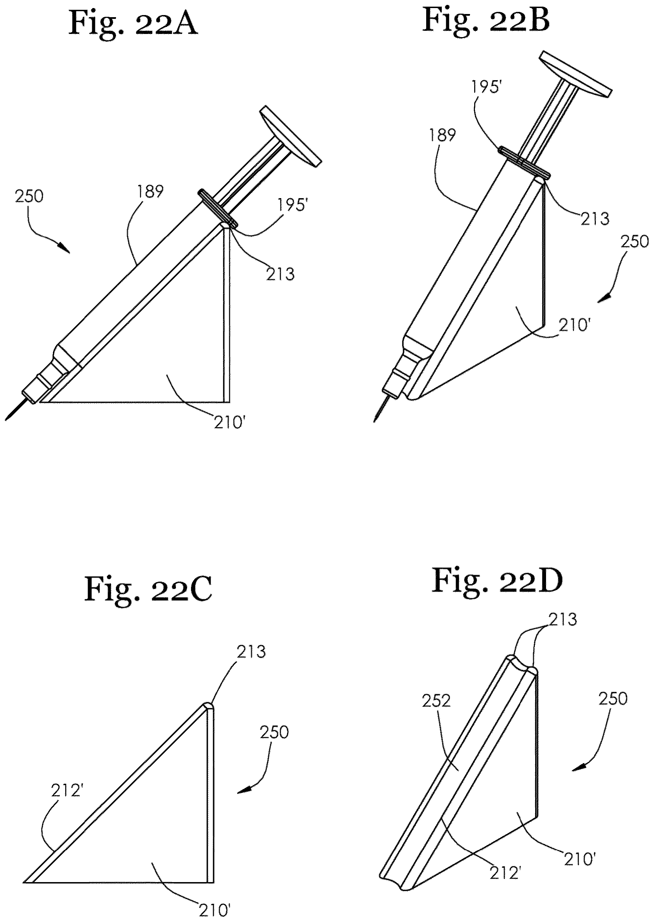

FIGS. 22A-D show different views of another embodiment of the positioning apparatus for guiding an injection device relative to a target surface shown in FIGS. 6A-B, having a groove for receiving an injection device. FIG 22A shows a side view of the positioning apparatus for guiding an injection device having a groove with the injection device loaded onto the positioning apparatus. FIG. 22B shows a perspective view of the embodiment shown in FIG. 22A. FIG. 22C shows a side view of the positioning apparatus shown in FIG. 22A without the injection device loaded thereon, and FIG. 22D shows a side perspective view of the embodiment of the positioning apparatus of FIGS. 22A-C without the injection device loaded thereon.

### DETAILED DESCRIPTION

For the purposes of promoting an understanding of the principles and operation of the invention, reference will now be made to the embodiments illustrated in the drawings and specific language will be used to describe the same. The invention is defined by the appended claims.

It is to be noted that the terms "first," "second," and the like as used herein do not denote any order, quantity, or importance, but rather are used to distinguish one element from another. The terms "a" and "an" do not denote a limitation of quantity, but rather denote the presence of at least one of the referenced item. The modifier "about" used in connection with a quantity is inclusive of the stated value and has the meaning dictated by the context (e.g., includes the degree of error associated with measurement of the particular quantity). It is to be noted that all ranges disclosed within this specification are inclusive and are independently combinable.

The terminology used herein is for the purpose of describing particular embodiments only and is not intended to be limiting. As used herein, the singular forms "a," "an," and "the" are intended to include the plural forms as well, unless the context clearly indicates otherwise these terms do not denote a limitation of quantity, but rather denote the presence of at least one of the referenced item. Furthermore, to the extent that the terms "including," "includes," "having," "has," "with," or variants thereof are used in either the detailed description and/or the claims, such terms are intended to be inclusive in a manner similar to the term "comprising." Moreover, unless specifically stated, any use of the terms first, second, etc., does not denote any order, quantity or importance, but rather the terms first, second, etc., are used to distinguish one element from another.

Notwithstanding that the numerical ranges and parameters setting forth the broad scope are approximations, the numerical values set forth in specific non-limiting examples are reported as precisely as possible. Any numerical value, however, inherently contains certain errors necessarily resulting from the standard deviation found in their respective measurements. Moreover, all ranges disclosed herein are to be understood to encompass any and all sub-ranges subsumed therein. As a non-limiting example, a range of "less than 10" can include any and all sub-ranges between (and including) the minimum value of zero and the maximum value of 10, that is, any and all sub-ranges having a minimum value of equal to or greater than zero and a maximum value of equal to or less than 10, e.g., 1 to 7.

### Definitions

The term "attachment component" as provided herein includes, but is not limited to an adhesive, a structural attachment means such as a gripping device that may be associated with or wrapped around a portion of a user to position the positioning apparatus, housing, and/or injection device against a target surface of the user. In one non-limiting example, the attachment component may include a bracelet-type structural device, which may be removably affixed to a user by placing around a limb of a user during an injection event. In another non-limiting embodiment, the attachment component may include an adhesive material associated with a portion of the stabilizing surface, configured to attach the positioning apparatus to a target surface of a user.

The term "visual indicator" as provided herein includes, but is not limited to, a marking such as an etching or a sticker or other type of marking known to those skilled in the art on a portion of the housing, or a light indicator to guide a positioning of an injection device relative to the housing, in one non-limiting example. In another, non-limiting embodiment (as shown in FIG 6A-B), the visual indicator may include a level-component or other such device for guiding an alignment of the injection device relative to the target surface. The level-component may be disposed on a surface of the housing to guide a user in positioning the apparatus relative to the target surface at the first angle, wherein at the first angle, a bubble may be disposed in the center of the level-component.

The term "about" as used herein includes a 5-10% or 5-10 degree variance.

The term "representative plane" as used herein in reference to the target surface includes a tangential plane to a normal on the target surface. For illustration purposes, in one example, a representative plane is a tangential plane to the normal of the insertion point on the target surface. that is an average taken from multiple varying levels of the representative surface, for example, quite often a patient tissue target surface does not lie in one plane. Consequently, the representative plane includes an average plane of the target surface.

The term "injection device" as used herein includes a needle-containing or a non-needle containing device. Injection device includes medicament delivery devices as well as training devices, and any combination of the two.

The term "condition" as used herein includes but is not limited to a user input, a status of the housing, positioning apparatus and/or injection device, anything that is sensed by the housing, positioning apparatus and/or injection device, correct or incorrect stepwise activities, usage of the housing, positioning apparatus and/or injection device, usage of the housing, positioning apparatus and/or injection device over time, among other conditions. The term "condition" is configured o include an "error condition", which includes but is not limited to a condition pertaining to a mistake by the user in using the housing, positioning apparatus and/or injection device, whether the mistake is incorrect positioning or contact between the housing, positioning apparatus and/or injection device and the user, or whether the mistake is an out of order step, a step that exceeds or fails to meet predetermined time value (such as an undue pause during or between steps, or insufficient time for conducting a step or transition between steps). Error conditions may also include errors of the housing, positioning apparatus and/or injection device, including low or lack of power or failure to operate as intended.

The term "associated" or "association", as used herein, includes but is not limited to direct and indirect attachment, adjacent to, in contact with, partially or fully attached to, and/or in close proximity therewith. The term "value" as used herein, may refer to a specific value or a range of values.

A "predetermined value" as used herein, for example, includes but is not limited to a value or range of values relating to an event involving use or operation of the housing, positioning apparatus and/or injection device. These may include, but are not limited to thresholds, ceilings, baselines or range values that are desired or undesired for a particular event. Examples of predetermined values include, but are not limited to, a predetermined orientation value, predetermined time value, or a predetermined contact value, in a particular example, predetermined value may refer to a predetermined angle value as described herein, in addition to other predetermined values described herein refers to a value that is used as a reference value in relation to a value, signal, or indication that is detected by, for example, a sensor of the housing, positioning apparatus and/or injection device. Predetermined value may include an optimal value, or a sub-optimal value, or any value there between.

In one non-limiting example, a predetermined value may include a 90 degree angle between the injection device, positioning apparatus and/or housing, and a target region for the injection, an additional predetermined value may include a 10 degree angle between the device and a target region for the housing, positioning apparatus and/or injection device, for example. At either predetermined value, or at any value there between, a signal output component may be initiated. The signal output component may therefore be an error message or a congratulatory message, for example.

The term "use" as used herein includes, but is not limited to, operation of the housing, positioning apparatus and/or injection device.

As used herein, the terms "subject" , "user" and "patient" are used interchangeably. As used herein, the term "subject" refers to an animal, preferably a mammal such as a non-primate (e.g., cows, pigs, horses, cats, dogs, rats etc.) and a primate (e.g., monkey and human), and most preferably a human.

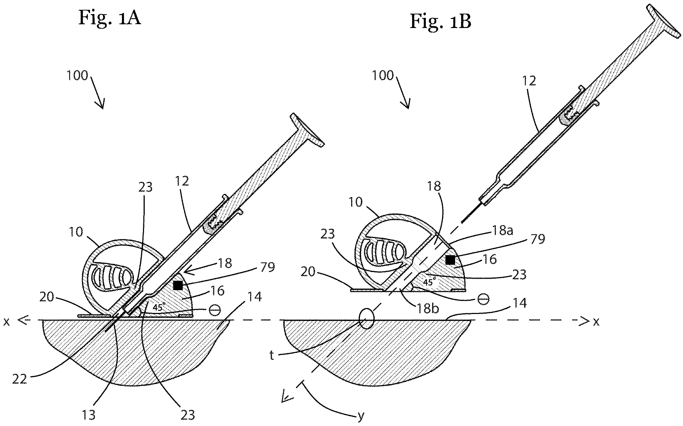

Turning to the Figures, FIGS. 1A-1B include side views of an embodiment 100 of a positioning apparatus 10 for guiding an injection device 12 relative to a target surface 14 having a representative plane x, the apparatus 10 includes a housing 16 defining a channel 18 configured to receive an injection device 12, wherein a first angle θ is formed between a longitudinal axis of the channel y and the representative plane x of the target surface 14 so as to guide the injection device 12 through the channel 18 to the target surface 14 at the first angle θ, and wherein the housing 16 comprises a stabilizing surface 20 configured to abut the target surface 14 to stabilize an injection according to the first angle θ. In a non-limiting embodiment, the first angle θ may include about a 90 degree angle. In another non-limiting embodiment, as shown in FIG. 1A-B, the first angle may include about a 45 degree angle, such the positioning apparatus 10 may facilitate an injection at the 45 degree angle relative to the target surface 14. FIG. 1A provides a side view in which the injection device 12 is inserted into the channel 18 of the housing 16 of the positioning apparatus 10, such that a needle 13 of the injection device 12 traverses the target surface 14 at a 45 degree angle. In one embodiment, as shown in FIG. 1A, the positioning apparatus 10 may further include an aperture 22 formed in the stabilizing surface 20 of the housing 16 adjacent to the target surface 14, wherein when the injection device 12 is received within the channel 18, a portion of a distal end of the injection device 12 aligns with the aperture 22 in the housing 16, such that the needle 13 of the injection device 12 can exit the housing 16, traversing the housing 10 through the aperture 22 and entering the target tissue at the first angle θ.

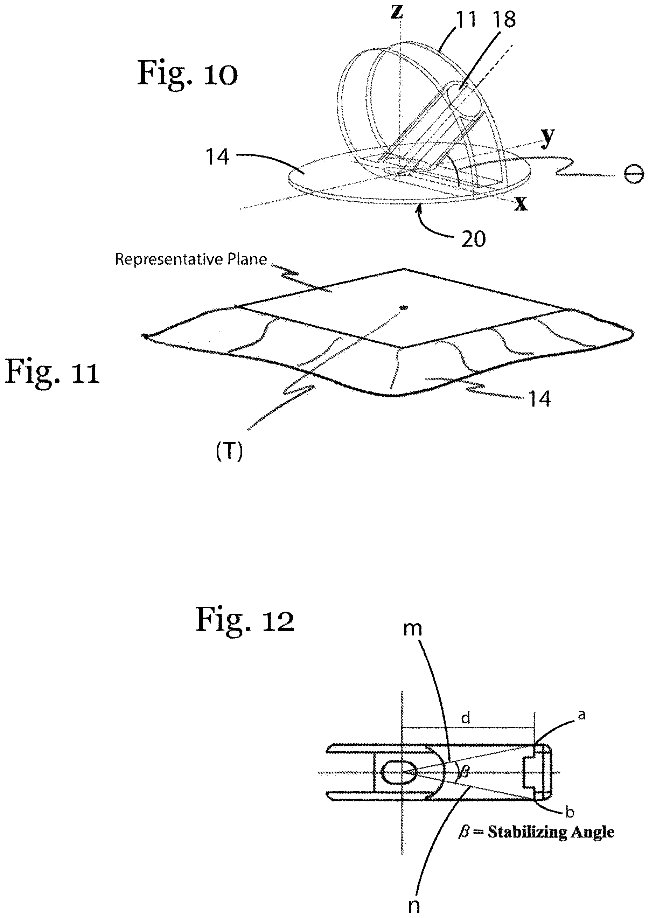

FIG. 1B provides a view in which the injection device 12 has not been placed within the apparatus 10, and the apparatus 10 is not against the target surface 14. In further non-limiting embodiments, the first angle may include an angle between about 10-15 degrees, an angle less than 90 degrees, an angle between about 40 and 50 degrees, or an angle between 30 degrees and 90 degrees, for example. The stabilizing surface 20 reduces pivoting of the longitudinal axis y of the channel 18 relative to the target surface 14. The channel 18 includes a proximal end 18a, a distal end 18b, and the stabilizing surface 20 is configured to contact the target surface 14 at at least two points (a, b), at least 1-5 cm from a center point of a distal opening of the channel and in at least two non-parallel linear axes, in one non-limiting embodiment (see FIG. 12). In a further non-limiting embodiment, the linear axes form a stabilization angle β there between that is at least 20 degrees (see FIG. 12). In a further embodiment, the positioning apparatus 10 is provided wherein the stabilizing surface 20 contacts the target surface 14 at at least three nonlinear points, a, b, c, at least 1-5 cm from a center point of a distal opening.

In a further embodiment, the housing 16 may include one or more protrusions 23 adjacent to and/or extending into the channel 18. The one or more protrusions 23 are configured to retain the injection device 12 within the housing 16 when the injection device 12 is received within the channel 18.

In one non-limiting embodiment, the injection device 12 may include an injection training device. In another non-limiting embodiment, the injection device 12 may include a medicament delivery device, wherein the channel 18 is configured to guide the medicament delivery device to deliver an injection at a target point t at the target surface 14, such that a longitudinal axis of the medicament delivery device y is determined by the longitudinal axis of the channel 18, and the medicament delivery device contacts the target surface 14 at the first angle θ.

In a further embodiment (as shown in FIG. 10), the positioning apparatus 11 may include a platform 25 extending from the stabilizing surface 20, the platform 25 may include a longitudinal axis transverse to the longitudinal axis of the channel 18, and parallel to the longitudinal axis x of the target surface 14, wherein the platform 25 is configured to abut the target surface 14.

In still a further embodiment, the platform 25 may included a first portion and a second portion, said first and second portions are disposed on either side of the housing 16, and the platform 25 comprises a flexible material such that a user can grip the first and second portions of the platform to receive at least a portion of the target surface 14 between the first and second portions of the platform 25 to displace the portion of the target surface 14 to receive the injection device 12. The flexible material may include, but is not limited to a rubber, a silicone, or any other deformable material known to those skilled in the art to which the invention pertains.

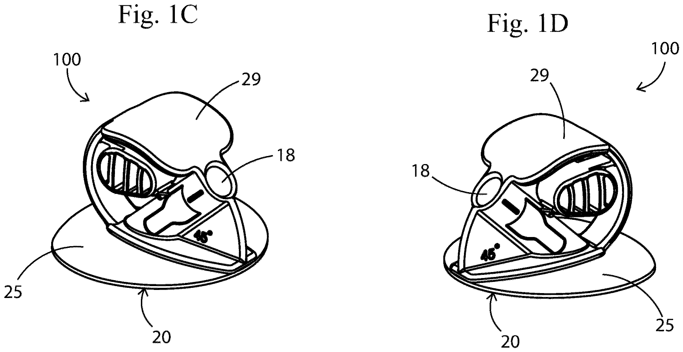

FIGS. 1C and 1D are perspective views of the positioning apparatus 100 showing the stabilizing surface 20 with an optional platform 25 extending outward from the stabilizing surface. The platform 25 may provide additional support to a user of the apparatus and may extend the stabilizing surface to further stabilize the apparatus during use. As described above, the platform 25 is configured to abut the target surface when the apparatus is in use. Furthermore, as described in some embodiments herein, the platform may optionally be made of a flexible material to allow a user to grip the area around and/or including the target area to provide an injection into the target area during use of the apparatus. The embodiment 100 may optionally include a safety guard 29 as shown in FIGS. 1C-1D, wherein the safety guard 29 may be provided to protect a user from needle prick during use of the apparatus, in one non-limiting embodiment. The safety guard 29 may also serve to visually guide the user to insert a portion of the injection device 12 (not shown in FIGS. 1C-1D), i.e., an injection member, into the channel 18 in a non-limiting embodiment.

Various safety features may be provided herein in regard to the positioning apparatus. One such safety feature includes the safety guard 29, wherein the portion of the user's hand that manipulates the positioning apparatus is protected from needle sticks from an injection device via the safety guard 29. At any point where a needle may come in contact with a user outside of the injection site, protection of the user is considered. Embodiments of the positioning apparatus described throughout this description also provide structural features for guiding an injection device via, in non-limiting examples, a visual guide, a channel within which the injection device may be received, or in another non-limiting example, walls of a positioning apparatus that prevent lateral movement and provide a lateral guide of the injection device during use, reduce or prevent risk of needle sticks during use of the apparatus with a needle-containing injection device.

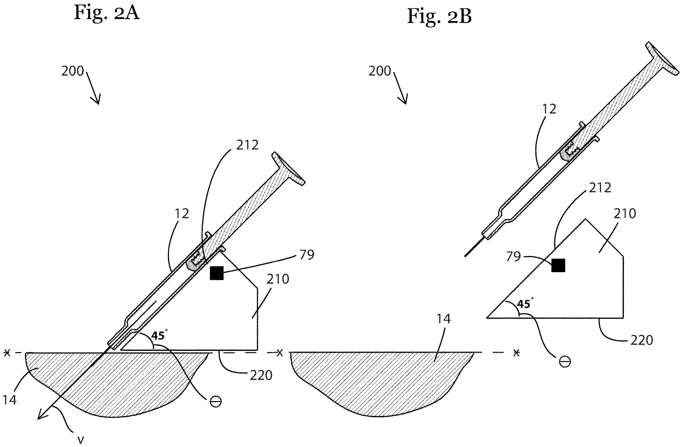

FIGS. 2A-2B include side views of an embodiment 200 of a housing 210 for guiding an injection device 12 relative to a target surface 14. The embodiment 200 includes a housing 210 having a support surface 212 for supporting and guiding the injection device 12, wherein the housing 210 is configured to facilitate control of the vector v of the injection device 12 relative to a target surface 14 having a representative plane x at a first angle θ relative to the representative plane x, wherein said housing comprises a stabilizing surface 220 configured to abut the target surface 14 to stabilize an injection at the target surface 14 according to the first angle θ.

In one embodiment, the vector v of the injection device 12 is less than a 90 degree angle relative to the representative plane x of the target surface 14. In a further embodiment, the vector v of the injection device 12 is between a 30 degree and a 90 degree angle relative to the representative plane x of the target surface 14. In another embodiment, the vector v of the injection device 12 is at about a 45 degree angle relative to the representative plane x of the target surface 14. In still another embodiment, the vector v includes about a 90 degree angle relative to the representative plane x of the target surface 14. In a further embodiment, the housing 210 may further include a platform portion 25 configured to abut a target surface 14. In still a further embodiment, the platform portion 25 may include a flexible material, the platform portion 25 having a first portion and a second portion, such that the platform 25 may be deformed around a portion of the target surface 14 received between the first portion and the second portion of the platform portion 25. The platform portion 25 may therefore be used to grip a layer of the target tissue between the first portion and the second portion of the platform 25 for receiving an injection in the target tissue.

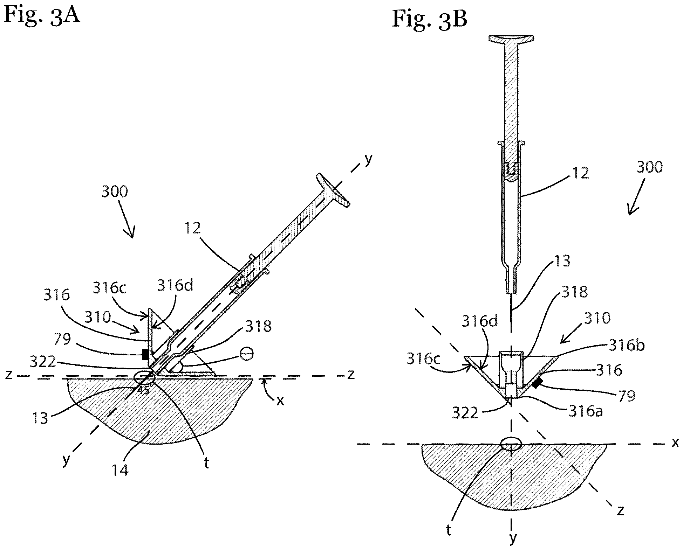

FIGS. 3A-3B include side views of a further embodiment 300 of a positioning apparatus 310 for guiding an injection device 12 relative to a target surface 14. The apparatus 310 includes a housing having a body 316 having a generally conical shape, the body 316 comprising a base portion 316b, an apex portion 316a, an outer surface 316c and an inner surface 316d, wherein an aperture 322 is defined at the apex portion 316a. A channel 318 disposed adjacent to the inner surface 316d, the channel 318 terminating at the aperture 322 in the apex portion 316a, the channel 318 is configured to receive an injection device 12, wherein a predetermined angle θ is formed between a longitudinal axis of the channel y and a longitudinal axis z of the outer surface 316c defined between the base portion 316b and the apex portion 316a of the body 316, such that when the outer surface 316c of the body 316 is positioned against the target surface 314 as shown in FIG. 3A, and an injection device 12 is placed in the channel 318, the injection device 12 contacts the target surface 14 through the aperture 322 at the predetermined angle θ. The predetermined angle θ in the non-limiting embodiment of FIG. 3A is 45 degrees.

When the body 316 of the positioning apparatus 310 is placed against the target surface 14, such that the outer surface 316c abuts the target surface 14, the longitudinal axis z of the outer surface 316c is generally aligned with the longitudinal axis of the representative plane x of the target surface 14 in the non-limiting embodiment of FIG. 3A. Consequently, the predetermined angle θ is formed between the longitudinal axis of the channel y and both the representative plane x of the target surface 14 and the longitudinal axis of the outer surface z in FIG. 3A. In one embodiment, the predetermined angle θ includes an angle less than 90 degrees. In another embodiment, the predetermined angle θ includes an angle between about 30 degrees and 90 degrees. In yet another embodiment, the predetermined angle θ is about 45 degrees as shown, for example in FIG. 3A.

The aperture 322 may be configured such that when the injection device 12 comprises a needle 13, the needle 13 may pass through the aperture 322 to a target point t on the target surface 14. The predetermined angle θ may include about a 90 degree angle, about a 45 degree angle, or about a 10-15 degree angle in non-limiting embodiments. In some examples, a 45 degree angle is used for subcutaneous injections. In other examples, a 45 degree angle may be used for intramuscular injections. In some instances a 90 degree angle is used during an injection with an auto-injector device, for example. FIG. 3B shows an embodiment in which the predetermined angle θ is about 90 degrees. Therefore, for example, in FIG. 3B, the predetermined angle θ is formed between the longitudinal axis of the channel y and the representative plane x of the target surface 14. In this non-limiting embodiment, the positioning apparatus 310 may be positioned against a target surface 14 at the target point such that a least a portion of its apex portion 316a contacts a portion of the target surface 14. In other non-limiting embodiments, for intra-dermal injections, for example, a 10-15 degree angle between the longitudinal axis of the injection device and the target surface is often preferred.

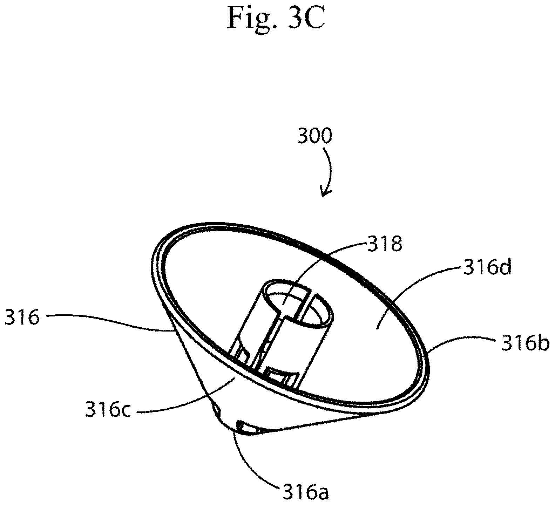

FIG. 3C is a perspective view of the embodiment 300 of the positioning apparatus shown in FIGS. 3A-3B. The embodiment 300 shown in FIG. 3C provides the body 316, having an apex 316a, a base 316b, an inner surface 316d and an outer surface 316c. The embodiment 300 also includes the channel 318 shown in FIG. 3C. When placed such that the base 316b is in contact with a target area of a user, as described above, an injection device 12 (not shown in IFG. 3C) can be received within the channel 318 to provide an injection or a simulated injection to a user at the target area, in one non-limiting embodiment. A predetermined angle θ formed between the injection device 12 and the surface of the user at the target area via the positioning apparatus 300 may include about a 45 degree angle, or about a 10-15 degree angle in non-limiting embodiments, depending on the size of the body 316, and the structural characteristics of the body 316, in a non-limiting example. The embodiment 300 may be placed, alternatively, such that the apex 316a abuts the target area t of the user (in the position shown in FIG. 3B when the apparatus is against the target point, t), and in this embodiment, the predetermined angle formed between the injection device 12 (not shown in FIG. 3C) when received within the channel 318, may include about a 90 degree angle between the injection device 12 and the target surface of the user (i.e., between the x and y axes as shown in FIG. 3B).

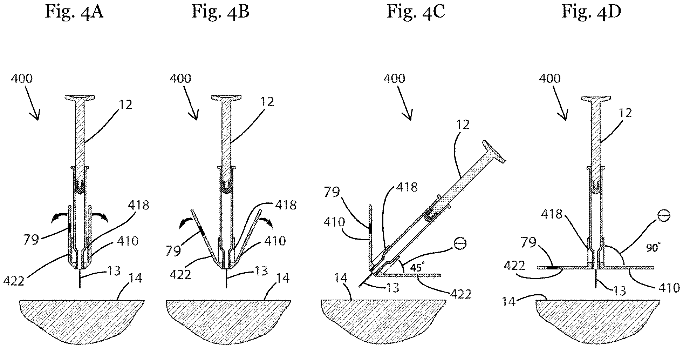

FIGS. 4A-4D include side views of still a further embodiment 400 of a positioning apparatus 410 for guiding an injection device 12 relative to a target surface 14. The positioning apparatus 410 includes a housing having an adjustable stabilizing surface 422, in one embodiment. The stabilizing surface 422 may include one or more components, which may be movable relative to a channel 418 of the positioning apparatus 410, to adjust a first angle θ formed between the channel 418 and the stabilizing surface 422. At least a portion of the stabilizing surface 412 is configured to abut a target surface 14 of a target tissue of a user, and the channel 418 is configured to receive an injection device 12 to guide the injection device 12 at the first angle θ to a target point t at the target surface 14 during an injection. The stabilizing surface 412 may be positioned such that the first angle θ includes a 45 degree angle as shown in FIG. 4C, in one non-limiting embodiment, or a 90 degree angle in another non-limiting embodiment as shown in FIG. 4D. Before and after use of the positioning apparatus 410, the stabilizing surface 422 may be positioned adjacent to the channel 418 as shown in FIG. 4A for storage and/or travel, for example. In one non-limiting embodiment, the positioning apparatus 410 may be affixed or attached to the injection device 12, or a component thereof.

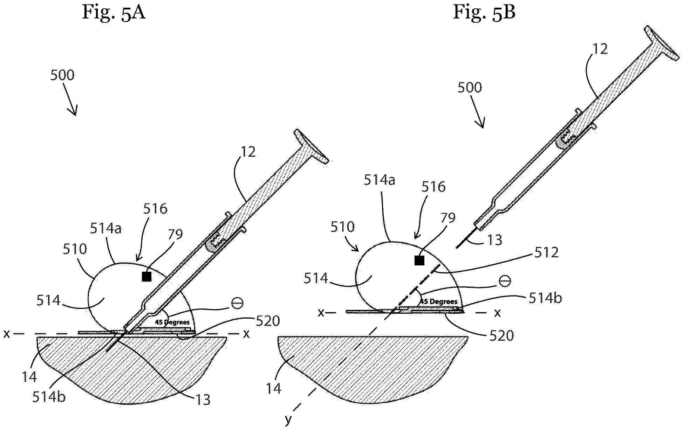

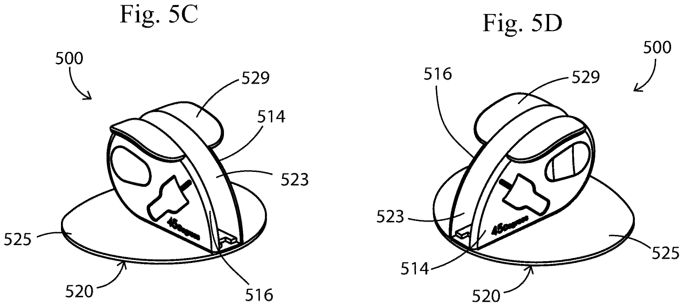

FIGS. 5A-5B include side views of another embodiment 500 of a positioning apparatus 510 for guiding an injection device 12 relative to a target surface 14, wherein the apparatus 510 includes a housing having a first side 514 having an upper edge portion 514a and a lower edge portion 514b, a second side 516 (not shown in FIG) having an upper edge portion 516a and a lower edge portion 516b, and a stabilizing surface 520 having a first edge portion 520a and a second edge portion 520b, wherein said first edge portion 520a of the stabilizing surface 520 associates with the lower edge portion 514b of the first side, and the second edge portion 520b of the stabilizing surface 520 associates with the lower edge portion 516b of the second side defining an opening 523 there between, and a visual indicator 512 on the first and/or second side 514, 516, wherein the visual indicator 512 may include a marking on the housing. The marking defines an angular placement of an injection device 12 relative to a longitudinal axis x of the stabilizing surface 520, such that when aligned with the marking, a first angle θ is formed between a longitudinal axis of the injection device y and the longitudinal axis of the stabilizing surface x.

In an embodiment, the stabilizing surface 520 is configured for placement on a patient against the target surface 14 for an injection. Placement of the injection device 12 in alignment with the visual indicator 512 guides the injection device 12 through the housing to the target surface 14 at the first angle θ. In a further embodiment, the stabilizing surface may include one or more apertures 522 for receiving an injection member 13 (i.e., a needle) of an injection device 12. In an embodiment, at least one of the one or more apertures 522 is disposed in the stabilizing surface 520 adjacent to the longitudinal axis of the injection device y when aligned with the visual indicator 512, the aperture 522 is configured to be associated with the target surface 14 and configured to receive the injection member 13 of the injection device 12. The injection device 12 may include an injection training device and/or an injection medicament delivery device. In a further embodiment, the stabilizing surface 520 may include a platform 525 configured to be positioned against a target surface 14 of a user. In a further embodiment, the platform may include a flexible material wherein a user can grip a portion of the target surface 14 between the flexible material of the platform 525 to raise the target surface 14 to receive the injection member 13. In one embodiment, the first angle may include about a 90 degree angle. In another embodiment, the first angle may include about a 45 degree angle. In still another embodiment, the first angle may include between about a 10-15 degree angle.

FIGS. 5C-5D include perspective views of the embodiment of the positioning apparatus 500 shown in FIGS. 5A-5B. The embodiment 500 includes the first side 514 and second side 516, the stabilizing surface 520 for placement against a user and an opening 523 between the first and second sides 514, 516 respectively, for receiving at least a portion of an injection device 512 (not shown). An optional safety guard 529 is shown in FIGS. 5C-5D, wherein the safety guard 529 protects a user from a potential needle stick when using the positioning apparatus with an injection device 12 having a needle, in an embodiment. The safety guard 529, also allows a user to grip onto the apparatus to position it against the target surface. Further, an optional platform 525 is shown extending from the stabilizing surface 520 of the embodiment 500. As described in reference to other embodiments, this platform 525 may further provide stabilization of the apparatus on the target surface of the user during use of the apparatus. Furthermore, the platform 525 may include a flexible material, such that it may be used by the user of the apparatus to grip a portion of the surface of the user surrounding the target area during use of the apparatus. In one non-limiting example, the platform 525 may be used to grip skin of the user surrounding or including the target area during an injection to better position a needle or injection member of the injection device 12 (not shown in FIGS. 5C-5D) into the target area, and to provide a more efficient injection into the target layer of the user (whether subcutaneous, intradermal, or other layer of the target area) as needed for effective deposition of the medicament contained within the injection device 12 for optimal therapy.

FIGS. 6A-6B include side views of one non-limiting embodiment of a visual indicator. The visual indicator may include a level-component 19 as shown in FIGS. 6A-B. The level component 19 may be provided to guide an injection device 12 to a target surface 14 at a predetermined angle θ. The level component 19 may be such that when an air bubble 30 is disposed in the center portion of the level-component 19, the correct predetermined angle θ between the injection device 12 and the target surface has been reached.

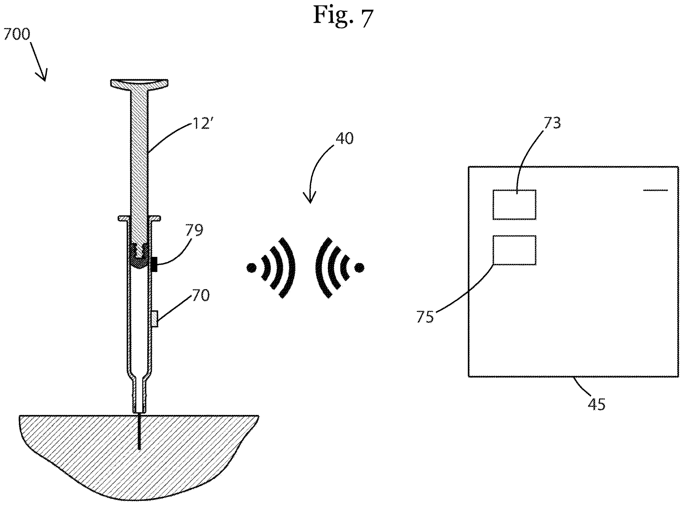

FIG. 7 includes a side view of a sensor embodiment of a positioning apparatus in wireless communication 40 with an external device 45. The external device 45 is an optional feature in this embodiment. The sensor embodiment 700 may include an electronic version of the positioning apparatus, having a position sensor 70, which may be embodied as an accelerometer or a tilt/angle sensor in non-limiting embodiments. There may further be an electronic chip on the injection device 12', and communications with an external device 45 as shown in FIG. 7, or with the user directly by way of a feedback feature using visual, audio or other cues stimulating other senses to provide information to a user and communicate therewith. For example, based on information obtained by the sensor, the positioning apparatus may communicate to the user whether the angle at which an injection device is being positioned is correct or incorrect. This can be accomplished by any methods known in the art, including, for example, by providing a visual feedback by the user regarding the angle by way of either the external device 45 and/or an onboard feedback features via a signal output component 73, such as a light indicator or a speaker on the injection device, in non-limiting examples. Consequently, the positioning apparatus can detect correct or incorrect positioning and/or angle of the injection device and provide feedback to a user based on the detection, or communicate with the external device 45 via component 75 the information obtained, by sending a signal to the external device 45 from the injection device 12' in one non-limiting embodiment.

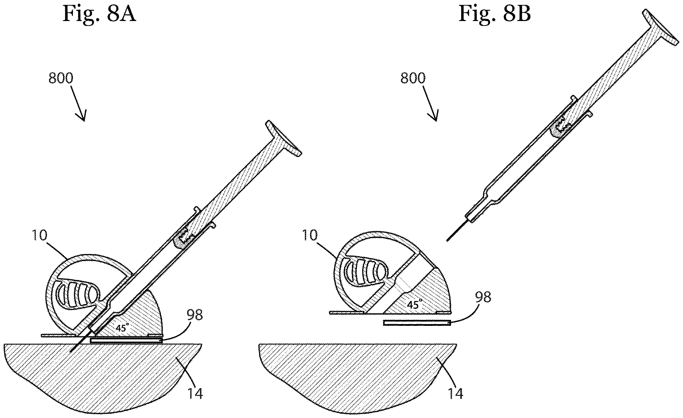

FIG. 8A-B shows side views of a non-limiting embodiment of a positioning apparatus including a housing 10 and an attachment component 98. The attachment component 98 is shown, in the non-limiting embodiment presented, as associated with a lower portion of the housing 10. The attachment component 98 can be adhered onto the target surface 14 of a user during use of the positioning apparatus, for example.

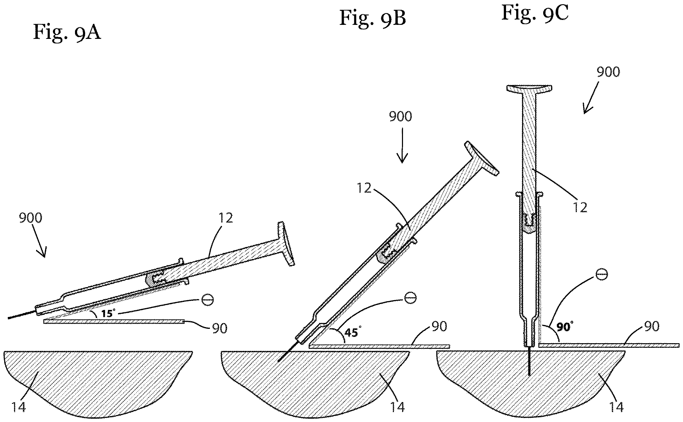

FIG. 9A-C includes side views of a non-limiting adjustable positioning apparatus embodiment 900, having a housing 90 which can be associated with a target surface 14 and an injection device 12 can be guided thereon for an injection at the target surface 14. The housing 90 may include a predetermined angle θ which may be adjustable as shown in FIGS. 9A-C. The angles shown are shown for example, only, and are not intended to be limiting of the embodiment provided herein.

FIG. 10 provides a non-limiting embodiment of a positioning apparatus 11 having a channel 18, a stabilizing surface 20, a predetermined angle θ, and a platform portion 25 extending from the stabilizing surface 20, and at least a portion of the platform portion 25 being configured to abut or associate with at least a portion of the target surface 14.

FIG. 11 provides an illustration of an example of a target surface 14 having a target point (T), showing a non-limiting example of a representative plane on the target surface 14.

FIG. 12 provides a bottom view of a non-limiting embodiment of a positioning apparatus providing at least two points (a, b,) which are configured to contact the target surface 14 at at least two points (a, b,), at a distance (d) of least 1-5 cm from a center point of a distal opening of the channel 18 and in at least two non-parallel linear axes (m, n). A stabilizing angle β is shown in FIG. 12, which is defined by the non-parallel linear axes. FIG. 12 is provided for illustration purposes to explain the stabilizing angle. Embodiments with a stabilizing surface that contacts target surface at different points, which would also include a broad stabilizing surface laying on the target surface at numerous points, would still nonetheless meet the criteria of having at least two points that contact the target surface.

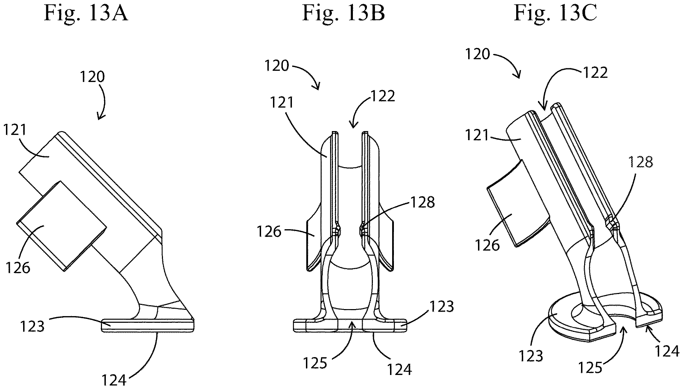

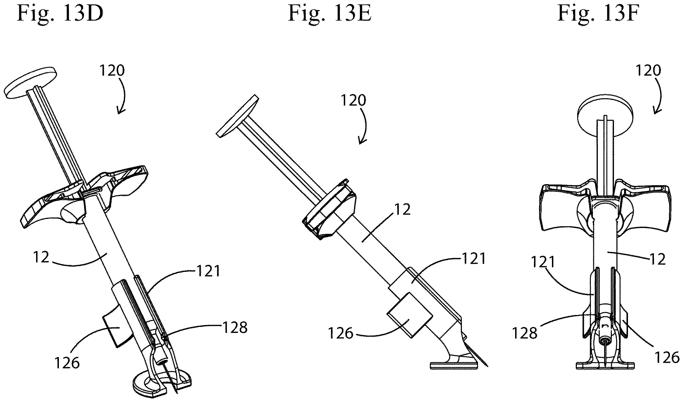

FIGS. 13A-13C provide side, front and perspective views of a further embodiment of a positioning apparatus 120 for directing or guiding an injection device to a target area for injection at a predetermined angle. The predetermined angle, in one non-limiting embodiment, may be formed between a stabilizing surface 124 and an angle of a channel 122 formed by a housing 121 of the positioning apparatus. The longitudinal axis of the channel 122 determines the angle at which the injection device 20 can be passed there through to the target surface of the user, in one non-limiting embodiment. In some non-limiting embodiments, this predetermined angle may be adjustable by a user. In other non-limiting embodiments, this angle may be fixed. The positioning apparatus 120 includes a housing 121 having a channel 122 terminating at an aperture 125 in a base 123 portion of the housing. The housing further includes a stabilizing surface 124. The positioning apparatus 120 further includes one or more tabs 126 for releasing the housing from an injection device 12 (not shown in FIGS 13A-C) associated with the channel 122 of the housing 121.

During use of the positioning apparatus 120, an injection device 12, shown in FIGS. 13D-13F, is placed into the channel 122, such that the housing 121 becomes removably affixed to the injection device 12, the base 123 with stabilizing surface 124 is placed against the target surface of the user, such that the aperture 125 aligns with the target point on the target surface of the user. The injection device 12 is actuated, and following use of the injection device 12 via the positioning apparatus 120, the positioning apparatus 120 is removed from the injection device 12 by manipulation of one or more tabs 126 to release the housing 121 from the device 12. In one non-limiting embodiment, two tabs 126 are provided, and compression of the tabs 126 toward one another releases the housing 121 from the injection device 12. In one non-limiting embodiment, the tabs 126 are used to separate the apparatus 120 from the injection device 12 following use, in order to provide a hands-free drop of the injection device into a sharps container, for example. This particular safety feature prevents contact with an exposed needle, in order to minimize risk of exposure and needle pricks. In the embodiment of the positioning apparatus 120, one or more protuberances 128 are provided on the housing 121. The protuberances 128, shown in FIGS. 13B, 13C, 13D, and 13F project into the channel 122, such that an injection device 20 having, for example, a rigid needle shield on its distal end can be inserted into the channel 122, and the protuberances 128 can separate the rigid needle shield from the injection device as the injection device is inserted into the housing 121 prior to an injection with the injection device 20, in one non-limiting example. In one non-limiting embodiment, the protuberances 128 provide sufficient separation between the rigid needle shield and the remainder of the injection device 20 that a user a can easily remove the rigid needle shield from the injection device 20 before use, while the injection device 20 is within the housing 121. In another non-limiting embodiment, the protuberances 128 may serve to completely remove the rigid needle shield from the injection device 20 as the injection device 20 is inserted into the housing 121 as shown in FIGS. 13D-13F (rigid needle shield is not shown).

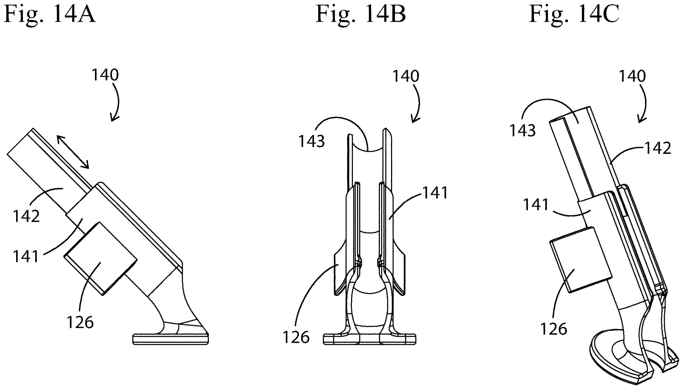

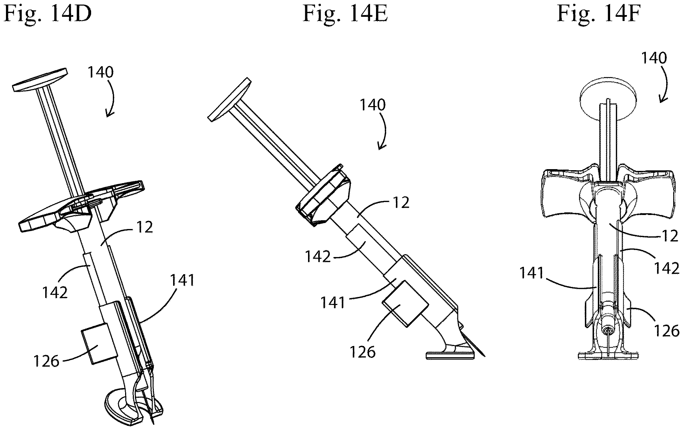

In a further embodiment shown in FIGS. 14A-14F, a positioning apparatus 140 in accordance with the invention is provided, wherein a guiding track 142 is associated with the housing 141 to guide an injection device 12 into the channel 143 of the apparatus 140. The guiding track 143 may also guide the injection device 12 along the longitudinal axis of the channel 143 and housing 141. The guiding track 142 may, in a non-limiting embodiment, move relative to the housing 141 to guide the injection device 12 toward the target surface of the user at a predetermined angle during use. In one particular non-limiting embodiment, the guiding track 142 may slide relative to the housing 141 in a first direction to deliver an injection device 12 to the target surface, and may slide in a second direction relative to the target surface to remove the injection device 12 from the target surface, for example.

In still a further embodiment, a positioning apparatus 150 for guiding an injection device relative to a target surface having a representative plane is provided. The apparatus 150 includes a housing 151 having a stabilizing surface 153, a guiding member 152 configured to receive and guide an injection device 12 (not shown) to a target surface at a first angle 156, wherein the housing 151 includes at least a first receiving member 154 and a second receiving member 155, the first and second receiving members 154, 155, being configured to surround a target area of the target surface during use. These receiving members 154, 155 may form a protective sleeve for the user to manipulate the positioning apparatus 150 at the target site for injection during use of the injection device. The receiving members 154, 155 may provide an added safety feature of protecting fingers of a user received within the receiving members 154, 155, in a non-limiting embodiment, from accidental needle prick by an injection device 12 (not shown) during use of the positioning apparatus with a needle-containing injection device, for example. In one non-limiting embodiment, at least a portion of the base portion of the housing 151 may include a flexible material, such that when a user inserts his or her fingers into the first and second receiving members 154, 155, and places the positioning apparatus 150 against the target surface, at least a portion of the target surface beneath the positioning apparatus 150 may be pinched between the first and second receiving members 154, 155, to target an injection at the target point when the injection device 12 is placed on the guiding member 152 and guided toward the target location.

In embodiments provided herein, a sensor 79 (as shown in FIG. 1A-1B, 2A-2B, 3A-3B) may be associated with the injection device 12 or the positioning apparatus, wherein the sensor 79 senses and records a use and/or a condition of the injection device 12 and/or of the positioning apparatus. One or more sensors may be incorporated into any of the embodiments described herein, not limited to the embodiments shown in FIGS. 1-3. In further embodiments, the sensor 79 may be configured to detect a condition or a use of the injection device 12 or the positioning apparatus based on a condition sensed by the sensor 79. In still a further embodiment, the sensor 79 may be configured to detect a condition of the housing and/or the injection device based on a condition detected by the sensor 79. In still a further embodiment, a condition may include an orientation, a position, a location, a temperature, a use, or a combination thereof, of the housing, positioning apparatus, and/or the injection device 12.

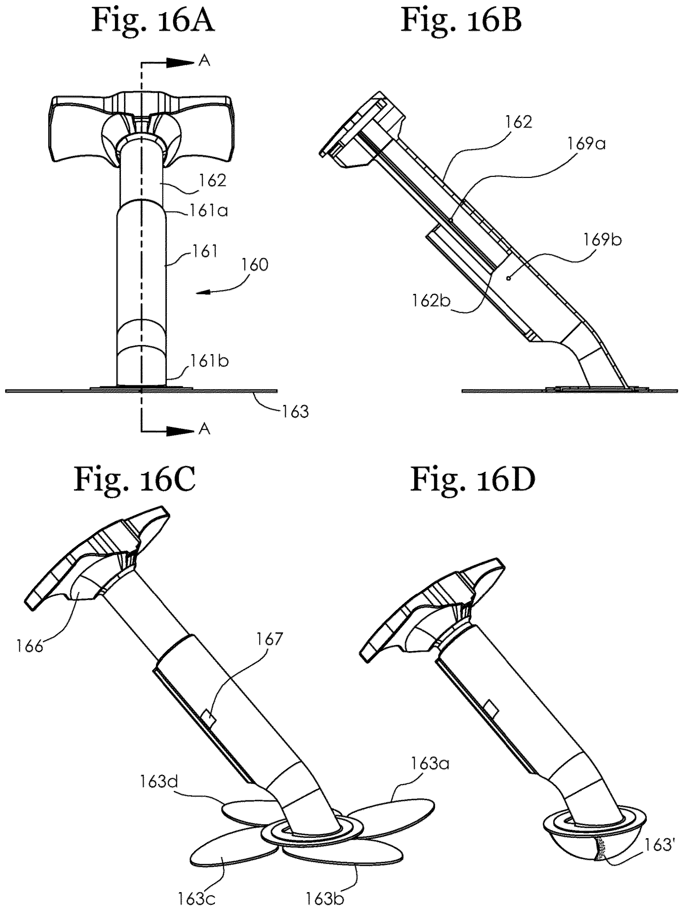

Turning to FIG. 16A-E, an injection assistive apparatus 160 is shown. The assistive apparatus includes a housing 161 and a guiding shuttle 162 that interacts with the housing 161. The housing 161 has a proximal end 161a and a distal end 161b. Associated with the distal end 161b is a base 163 that is placed on a target surface (not shown). The apparatus 160 includes a first lock mechanism 169a that holds the guide shuttle 162 in an unactuated state and a second lock mechanism 169b that holds the guiding shuttle 162 in an actuated state. Examples of a lock mechanism include, but are not limited to, a button on the guiding shuttle 162 or housing 161 that engages one or the other components. Another lock mechanism pertains to a detent member on the guide shuttle 162 or housing 161 that engages to one or the other components and provides a resistance force to hold the guiding shuttle in an unactuated or actuated state. The detect member may interact with a dimple on the opposing component.

The guiding shuttle 162 has a proximal end 162a and a distal end 162b. Associated with the proximal end is a bracket 166 that engages an injection device.

FIG. 16C shows that the base 163 includes a number of segments 163a, 163b, 163c, and 163d, that are unfolded in an open state. Those skilled in the art will appreciate that the base may include more or less segments than that shown. The segments 163a-d can fold into a closed state shown in FIG. 16D, whereby they serve to shield to a sharp component (e.g. needle) of an injection device. Associated with one or more of the segments 163a-d is one or more locking members 163' that hold the segments 163a-d in the closed state. Examples of such locking members includes a clip, snap, hook/look fabric, adhesive, etc. Provided on the apparatus 160 is one or more sensors and/or circuitry 167 to sense and monitor one or more aspects of operation or functionality of the apparatus 160.

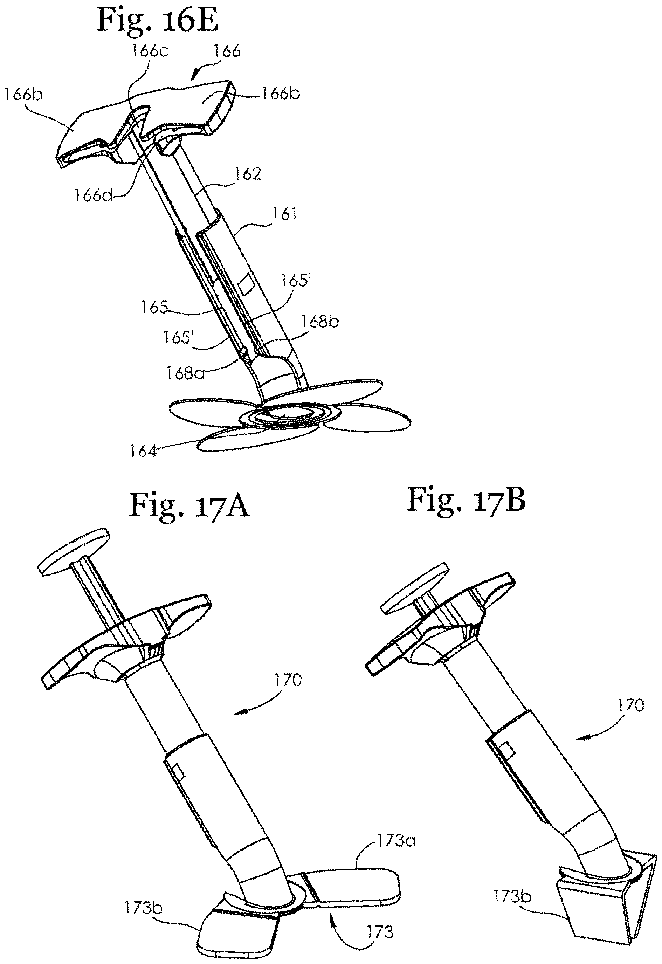

As shown in FIG. 16E, the bracket 166 includes flange members 166b and a central slot 166c for receiving an injection device. The bracket also includes a groove 166d for receiving an end piece 195' (see FIG. 18E) of the injection device. Further the housing 161defines a channel 165 and a longitudinal opening 165' for receiving an injection device. The guiding shuttle 162 may move relative to the housing 161 in a first direction toward a target surface (not shown) and in a second direction away from the target surface so as to guide the injection device (not shown) along the longitudinal axis A-A (FIG. 16A) of the channel 165. Disposed on the longitudinal opening 165' are protuberances 168a and 168b that are configured to interact with a rigid needle shield (RNS) of an injection device such that the RNS separates from the injection device as it is inserted into the channel. Further, the housing 161 The base 163 includes a base open region 164 that allows the injection device to pass through to the target surface.

FIG. 17A-B shows a side perspective view of an alternative injection assistive apparatus 170. The apparatus 170 includes a base 173 that has two segments 173a and 173b that move from an open state FIG. 17A to a closed state FIG. 17B. The segments may include a locking member as described above for FIG. 16.

Turning to FIG. 18A-I, an injection assistive apparatus 180 is shown. Apparatus 180 includes a spring mechanism that urges the apparatus from an actuated to an unactuated state as will be described further. Such mechanism makes apparatus 180 particular suitable for reuse. The assistive apparatus includes a housing 181 and a guiding shuttle 182 that interacts with the housing 181. The housing 181 has a proximal end 181a and a distal end 181b. The fab members 181c and 181d at the opening of channel 185 assist with guiding the device 189 (FIG. 18E) into the channel 185. Associated with the distal end 181b is a base 183 that is placed on a target surface (not shown). The apparatus 180 includes a first lock mechanism 189a that holds the guide shuttle 182 in an unactuated state and a second lock mechanism 189b that holds the guiding shuttle 182 in an actuated state. As noted above for apparatus 160, examples of a lock mechanism include, but are not limited to, a button on the guiding shuttle 182 or housing 181 that engages one or the other components. Another lock mechanism pertains to a detent member on the guide shuttle 182 or housing 161 that engages to one or the other components and provides a resistance force to hold the guiding shuttle in an unactuated or actuated state. The detent member may interact with a dimple or hole (note shown) on the opposing component.

The guiding shuttle 182 has a proximal end 182a and a distal end 182b. Associated with the proximal end is a bracket 186 that engages an injection device.

The base 183 may or may not include segments as described above for apparatuses 160 and 170. Shown in FIG. 18G the base 183 pertains to a single body that defines a base open region 184. The base 183 may be flexible. Provided on a bottom side of the base 183 are a plurality of protrusions 197 that serve as pressure points to give neurological stimulus that ameliorates the sensation of needle entry.

The guiding shuttle 182 may move relative to the housing 181 in a first direction toward a target surface (not shown) and in a second direction away from the target surface so as to guide the injection device (not shown) along the longitudinal axis of the channel 185. The housing 181 includes a compartment 195 that holds a spring 196. The spring 196 abuts a spring tab 194 on the guiding shuttle 182. The spring 196 urges the guiding shuttle 182 from an actuated state (where the guiding shuttle is moved toward the distal end 182b such that the needle extends through the base open region 184) to an unactuated state where the guiding shuttle moves in the proximal direction away from a target surface.

As shown in FIG. 18A, the bracket 186 includes flange members 186b and a central slot 186c for receiving an injection device 189. The bracket also includes a groove 186d for receiving an end piece 195' of the injection device. Further the housing 181 defines a channel 185 and a longitudinal opening 185' for receiving an injection device 195. Disposed on the longitudinal opening 185' are protuberances 188a and 198b that are configured to interact with a rigid needle shield (RNS) of an injection device such that the RNS separates from the injection device as it is inserted into the channel.

FIG. 18F shows a cross-sectional view of the apparatus 180 and the injection device 189 (when inserted) along axis B-B indicated in FIG. 18A. Provided on the housing 181 are tabs 192a and 192b positioned such that squeezing the projections 192a,b together opens the longitudinal opening 185' slightly, which facilitates entry or exit of the injection device 195 into and out of the channel, respectively.

Shown in FIG. 19 is an alternative apparatus 190 that includes the features of the apparatus 180, but wherein the housing 191 has a pivot mechanism 191' that allows for adjustment of the angle 193. Accordingly, pivot mechanism 191' enables the user to adjust the angle at which injection is executed. It is noted that pivot mechanism can be implemented with other embodiments disclosed herein such as apparatus 160 or 170.

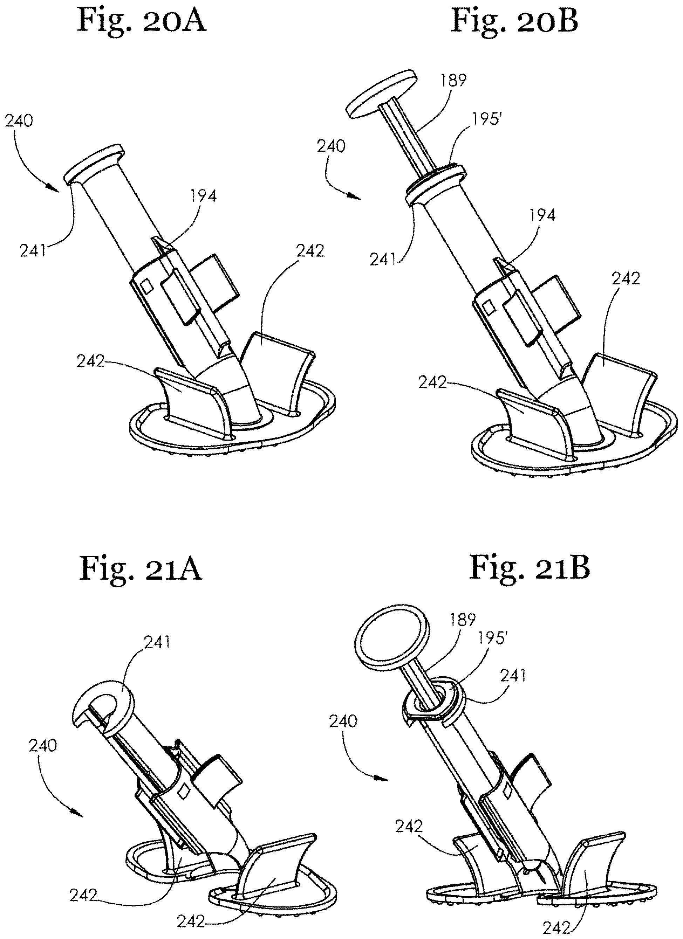

FIGS. 20A-B and 21A-B include an alternative apparatus 240 that includes the features of the apparatus 190 without the bracket 186. Furthermore, the apparatus 240 includes protective components 242. Protective components 242 are provided to interact with the fingers of a user during use of the apparatus 240 from needle sticks and from slipping of the fingers of the user during use of the apparatus 240. A portion of the protective components 242 may include a non-slip material which may contact the fingers of the user to prevent slipping of the fingers from the apparatus 190 during use. FIGS. 20B and 21B include the apparatus 190 shown with the injection device inserted therein, such that the end piece 195'abuts the stopping member 241, therein controlling the position of the injection device 189 relative to the apparatus 190, and consequently in some embodiments, controlling the depth of the needle of the injection device 189 relative to a target surface of a user.

In yet another embodiment, a positioning apparatus 250 is provided having a housing 210', a support surface 212', and a housing upper edge portion 213. The support surface 212' may include a groove 252 for receiving a portion of an injection device placed on the support surface 212' during use of the apparatus 250 as shown in FIGS. 22A-B. Groove 252 can be seen in FIG. 22D. The injection device 189 may be placed into the groove 252 of the support surface 212' of the apparatus 250 wherein the end piece 195' of the injection device 189 abuts the housing upper edge portion 213 as shown in FIGS. 22A-B. This interactivity between the end piece 195' ad the upper edge portion 213 prevents the injection device 189 from extending too far past a target position on the support surface 212'. This embodiment of the apparatus 250 is an alternative embodiment to the positioning apparatus embodiment 200 shown in FIGS. 2A-B. Also, a sensor can be provided on the housing 210' to detect a condition of the housing 210' and/or the injection device 189 based on a condition detected by the sensor.

Embodiments of the positioning and assisting apparatuses described herein may be configured to include structural features to control or adjust needle depth. For example, needle depth relative to a target area of a user may be controlled by the structural components of the embodiments described herein. In a non-limiting example, needle depth relative to a user of a needle of the injection device when the injection device is inserted into the assisting apparatus may be controlled whereby the end piece 195' of the injection device 189 abuts the stopping member 241 of the apparatus 240 as shown in FIGS. 20B and 21B. In another non-limiting embodiment, as shown in FIG. 16B, needle depth can be controlled by way of the first lock mechanism 169a and a second lock mechanism 169b of apparatus 160. In yet another non-limiting embodiment as shown in the apparatus 250, needle depth may be controlled by way of upper housing edge 213 upon interaction with the end piece 195' of the injection device 189 upon placement onto support surface 212', or by interaction between the flange of the injection device 12 and the upper portion of housing 210 shown in FIG. 2A. In one non-limiting embodiment, a combination of the longitudinal length of the support surface 212' and the injection device housing may determine the needle depth during use of the apparatus 250. In still a further non-limiting embodiment, shoulders of the injection device shown in FIG. 1A may interact with the protrusions 23 adjacent to and/or extending into the channel 18 of the apparatus 100 so as to control the depth of the needle of the injection device relative to the target surface 14. The needle depth may be controlled by varying these structures discussed herein to increase or decrease needle depth as needed.

In further embodiments, the positioning apparatus may be associated with an information sending, detecting and/or receiving component configured to receive information or send information about the injection device and/or the housing related to the condition, and optionally further comprising at least one of: a signal output component, a microprocessor, a storage medium component, and a power source. The term "circuitry" as used herein broadly refers to sensors and other electronic components related to sensing, recording, processing and/or transmitting information. In still a further embodiment, the positioning apparatus senses and records use of the positioning apparatus and/or the injection device. In yet a further embodiment, the signal output component is configured to output an audio, a visual, a tactile, a gustatory, or an olfactory signal, or a combination thereof, to a user.

In yet a further embodiment, the positioning apparatus may be configured to send information to and/or receive information from an external device via a wired or a wireless connection, wherein said connection provides a communication of power and/or information between the external device and the housing. The wireless connection may include a Bluetooth ^{®} and/or an RFID (Radio Frequency Identification) technology, in non-limiting embodiments. The RFID technology may include an RFID transponder and an RFID reader.

In another embodiment, the housing or the injection device further includes either a unique identification component, the unique identification component including information about a medicament, configured to be read by a unique identification reader associated with the injection device, or a unique identification reader configured to read information on a unique identification component associated with the injection device. In a further embodiment, the unique identification component may be a bar code and the unique identification reader is a bar code reader. In further embodiments, the positioning apparatus or injection device may include pre-programmed information about an injection device and/or a medicament. In still a further embodiment, the positioning apparatus, housing, or injection device may be configured to download or receive information from a database, the Internet, and/or another device, the information may include information about a medicament, information about an injection device including instructions for use, information about contraindication of medicaments, information about a user, medicament dosage information, storage information, prescribing physician information, pharmacy information, manufacturer information, warning information, recall information, environmental data, geographical information, time zone information, MET (meteorological) data, storage information, supply chain information, transit data, and/or temperature data.

In a further embodiment, the signal output component may include a speaker, a display, a light, a vibration component, a smell-emitting component, or a temperature-changing component, or a combination thereof. In still a further embodiment, the condition may include an error condition, a correct usage of the system, and/or an input sensed by the system. In y et a further embodiment, the positioning apparatus, an external device or the housing may provide a feedback to a user via a signal output component based on the condition detected. In still a further embodiment, the housing, positioning apparatus, injection device, or external device may provide instructions for using the injection device in a sequence of steps, and wherein the microprocessor is configured to control a provision of the instructions for using the injection device and/or the housing to the user in the sequence of steps and/or to provide an instruction to the user based on information detected and/or received by the information detecting and/or receiving component of the housing. In one non-limiting embodiment, the instructions may pertain to correct or incorrect angular positioning of the injection device relative to the target surface.

In a further embodiment, a visual indicator configured to provide correct angular alignment of the injection device relative to the target surface when the injection device is aligned with the visual indicator is provided. Alignment of the injection device with the housing or positioning apparatus as indicated by the visual indicator may provide correct alignment for proper angular alignment for an injection. In a further, non-limiting embodiment, the visual indicator may include a light and/or a marking on the housing and/or the injection device.

It is important to an understanding of the present invention to note that all technical and scientific terms used herein, unless defined herein, are intended to have the same meaning as commonly understood by one of ordinary skill in the art. The techniques employed herein are also those that are known to one of ordinary skill in the art, unless stated otherwise.

### CITED REFERENCES

[1] Zambanini, A., Newson, R.B.,, Maisey, M., Feher, M.D. (1999). Injection related anxiety in insulin-treated diabetes. Diabetes Research and Clinical Practice, 46, 239-246.
[2] Klobassa, N., Moreland, P. (2012, January 10). Psychological insulin resistance stems from fear. MayoClinic.com Living with Diabetes Blog, Message posted to http://www.mayoclinic.com/health/psychological-insulin-resistance/MY01165.
[3] Nam, S., Chesla, C., Stotts, N.A., et al. (2010). Factors associated with psychological insulin resistance in individuals with Type 2 diabetes. Diabetes Care, 33, 1747-1749.

## Claims

1. A positioning apparatus for guiding an injection device (12) relative to a target surface (14) having a representative plane, the apparatus comprising:
a housing (16) defining a channel (18) configured to receive an injection device (12), wherein a first angle (θ) is formed between a longitudinal axis (y) of the channel (18) and the representative plane (x) of the target surface (14) so as to guide the injection device (12) through the channel (18) to the target surface (14) at the first angle (θ), and wherein said housing (16) comprises a stabilizing surface (20) configured to abut the target surface (14) to stabilize an injection according to said first angle (θ);
wherein the injection device (12) comprises a medicament delivery device, and wherein the channel (18) is configured to guide the medicament delivery device to deliver an injection at a target point (t) at the target surface (14), such that a longitudinal axis of the medicament delivery device is determined by the longitudinal axis of the channel (18), and the medicament delivery device contacts the target surface (14) at the first angle (θ); and further comprising an aperture (22) formed in the stabilizing surface (20) of the housing (16) adjacent to the target surface (14), wherein when the injection device (12) is received within the channel (18), a portion of a distal end of the injection device (12) aligns with the aperture (22) in the housing (16), such that an injection device (12) comprising a needle (13) at its distal end can exit the housing (16) wherein the needle (13) traverses the housing (16) through the aperture (22);
the positioning apparatus further comprising a platform (25) extending from the stabilizing surface (20), the platform (25) comprising a longitudinal axis transverse to the longitudinal axis of the channel, wherein the platform (25) is configured to abut the target surface (14), wherein the platform (25) comprises a first portion and a second portion, said first and second portions are disposed on either side of the housing (16), and said platform (25) comprises a flexible material such that a user can grip the first and second portions of the platform (25) to receive at least a portion of the target surface (14) between the first and second portions of the platform to displace the portion of the target surface (14) to receive the injection device (12),
wherein the positioning apparatus further comprises a guiding track (142) associated with the housing (16), said guiding track (142) being configured to receive at least a portion of the injection device (12), wherein the guiding track (142) guides the injection device (12) along the longitudinal axis (y) of the channel (18), wherein the guiding track (142) is movable within the channel (18), relative to the housing (16), such that when the injection device (12) is received within the guiding track (142), the guiding track (142) is movable in a first direction toward the target surface (14) and in a second direction away from the target surface (14).

2. The positioning apparatus of claim 1, wherein the first angle (θ) comprises one of the following:
- an angle about a 90 degree angle;
- an angle between about a 10-15 degree angle or,
- an angle between about 40-50 degrees.

3. The positioning apparatus of claim 1, wherein the channel (18) has a proximal end (18a) and a distal end (18b), and wherein the stabilizing surface (20) contacts the target surface (14) at at least two points, at least 1-5 cm from a center point of a distal opening of the channel and in at least two non-parallel linear axes.

4. The positioning apparatus of claim 1, wherein the housing (16) comprises one or more protrusions (23) adjacent to the channel (18), said one or more protrusions (23) are configured to retain the injection device (12) within the housing (16) when the injection device (12) is received within the channel (18).

5. The positioning apparatus of claim 1, further comprising a sensor (79) associated with the housing, said sensor configured to detect a condition of the housing and/or the injection device based on a condition detected by the sensor.

6. The positioning apparatus of claim 5, wherein the positioning apparatus is associated with an information sending, detecting and/or receiving component configured to receive information or send information about the injection device and/or the housing related to a condition of the injection device and/or the housing, and optionally further comprises at least one of: a signal output component, a microprocessor, a storage medium component, and a power source.

7. The positioning apparatus of claim 1, wherein the positioning apparatus further comprises either a unique identification component, said unique identification component comprising information about a medicament, configured to be read by a unique identification reader associated with the injection device, or a unique identification reader configured to read information on a unique identification component associated with the injection device.

8. The positioning apparatus of claim 6, wherein the positioning apparatus is configured to download or receive information from a database, the Internet, and/or another device, said information comprises information about a medicament, information about an injection device including instructions for use, information about contraindication of medicaments, information about a user, medicament dosage information, storage information, prescribing physician information, pharmacy information, manufacturer information, warning information, recall information, environmental data, geographical information, time zone information, MET (meteorological) data, storage information, supply chain information, transit data, and/or temperature data.

9. The positioning apparatus of claim 6, wherein the signal output component comprises a speaker, a display, a light, a vibration component, a smell-emitting component, or a temperature-changing component, or a combination thereof.

10. The positioning apparatus of claim 6, wherein the positioning apparatus may provide instructions for using the positioning apparatus and/or the injection device, and wherein the microprocessor is configured to control a provision of the instructions for using the injection device and/or the positioning apparatus to a user and/or to provide an instruction to the user based on information detected and/or received by the information detecting and/or receiving component of the positioning apparatus.

11. The positioning apparatus of claim 1, wherein the guiding track (142) is slidable relative to the housing (16).

12. A method for positioning an injection device (12) at a target point (t) of a target surface (14) along a first angle (θ) relative to the target surface (14) having a representative plane (x), said method comprising:
positioning a positioning apparatus comprising a housing (16) having a guide and a guiding track (142) associated with the housing (16), the positioning apparatus being configured to receive an injection device (12), wherein the guide forms a first angle between a longitudinal axis of the guide and the representative plane of the target surface (14) so as to guide an injection device (12) to the target surface (14) at the first angle (θ), wherein the guiding track (142) is configured to receive at least a portion of the injection device (12) and is movable in a first direction towards the target surface (14) and in a second direction away from the target surface (14), and wherein said housing (16) comprises a stabilizing surface (20) to stabilize an injection at the target surface (14) according to said first angle (θ), such that the stabilizing surface (20) of the housing (16) abuts the target surface (14), and a distal portion of the guide is in alignment with the target point (t) of the target surface (14), the positioning apparatus further comprising a platform (25) extending from the stabilizing surface (20), the platform (25) comprising a first portion and a second portion, said first and second portions are disposed on either side of the housing (16), and said platform (25) comprises a flexible material such the first and second portions of the platform (25) can receive at least a portion of the target surface (14) between the first and second portions of the platform to displace the portion of the target surface to receive the injection device (12);
receiving a portion of the target surface (14) between the first and second portions of the platform (25);
placing the injection device (12) into the housing (16) in alignment with the guide at the first angle (θ); and
gliding the injection device (12) to the target point (t).

## Patentansprüche

1. Positioniervorrichtung zum Führen einer Injektionsvorrichtung (12) relativ zu einer Zielfläche (14) mit einer repräsentativen Ebene, wobei die Vorrichtung Folgendes umfasst:
ein Gehäuse (16), das einen Kanal (18) definiert, der dazu ausgebildet ist, eine Injektionsvorrichtung (12) aufzunehmen, wobei ein erster Winkel (θ) zwischen einer Längsachse (y) des Kanals (18) und der repräsentativen Ebene (x) der Zielfläche (14) ausgebildet ist, um die Injektionsvorrichtung (12) in dem ersten Winkel (θ) durch den Kanal (18) zu der Zielfläche (14) zu führen, und wobei das Gehäuse (16) eine Stabilisierungsfläche (20) umfasst, die dazu ausgebildet ist, an die Zielfläche (14) zu stoßen, um eine Injektion gemäß dem ersten Winkel (θ) zu stabilisieren;
wobei die Injektionsvorrichtung (12) einen Medikamentenabgabevorrichtung umfasst, und wobei der Kanal (18) dazu ausgebildet ist, die Medikamentenabgabevorrichtung so zu führen, dass sie eine Injektion an einem Zielpunkt (t) der Zielfläche (14) abgibt, sodass eine Längsachse der Medikamentenabgabevorrichtung durch die Längsachse des Kanals (18) bestimmt wird, und wobei die Medikamentenabgabevorrichtung die Zielfläche (14) in dem ersten Winkel (θ) berührt; und ferner umfassend eine Öffnung (22), die in der Stabilisierungsfläche (20) des Gehäuses (16) angrenzend an die Zielfläche (14) ausgebildet ist, wobei dann, wenn die Injektionsvorrichtung (12) in dem Kanal (18) aufgenommen ist, ein Abschnitt eines distalen Endes der Injektionsvorrichtung (12) mit der Öffnung (22) in dem Gehäuse (16) fluchtet, sodass eine Injektionsvorrichtung (12) mit einer Nadel (13) an ihrem distalen Ende aus dem Gehäuse (16) austreten kann, wobei die Nadel (13) durch die Öffnung (22) hindurch das Gehäuse (16) durchquert;
wobei die Positioniervorrichtung ferner eine Plattform (25) umfasst, die sich von der Stabilisierungsfläche (20) erstreckt, wobei die Plattform (25) eine Längsachse quer zur Längsachse des Kanals umfasst, wobei die Plattform (25) dazu ausgebildet ist, an die Zielfläche (14) zu stoßen, wobei die Plattform (25) einen ersten Abschnitt und einen zweiten Abschnitt umfasst, wobei der erste und der zweite Abschnitt auf beiden Seiten des Gehäuses (16) angeordnet sind und die Plattform (25) ein biegsames Material umfasst, sodass ein Benutzer den ersten und den zweiten Abschnitt der Plattform (25) greifen kann, um wenigstens einen Abschnitt der Zielfläche (14) zwischen dem ersten und dem zweiten Abschnitt der Plattform aufzunehmen, um den Abschnitt der Zielfläche (14) zu verschieben, um die Injektionsvorrichtung (12) aufzunehmen,
wobei die Positioniervorrichtung ferner eine mit dem Gehäuse (16) verbundene Führungsschiene (142) umfasst, wobei die Führungsschiene (142) dazu ausgebildet ist, wenigstens einen Abschnitt der Injektionsvorrichtung (12) aufzunehmen, wobei die Führungsschiene (142) die Injektionsvorrichtung (12) entlang der Längsachse (y) des Kanals (18) führt, wobei die Führungsschiene (142) in dem Kanal (18) relativ zu dem Gehäuse (16) bewegbar ist, sodass dann, wenn die Injektionsvorrichtung (12) in der Führungsschiene (142) aufgenommen ist, die Führungsschiene (142) in einer ersten Richtung zu der Zielfläche (14) hin und in einer zweiten Richtung von der Zielfläche (14) weg bewegbar ist.

2. Positioniervorrichtung nach Anspruch 1, wobei der erste Winkel (θ) einen der folgenden umfasst:
- einen Winkel von etwa 90 Grad;
- einen Winkel zwischen etwa 10 bis 15 Grad, oder
- einen Winkel zwischen etwa 40 bis 50 Grad.

3. Positioniervorrichtung nach Anspruch 1, wobei der Kanal (18) ein proximales Ende (18a) und ein distales Ende (18b) hat, und wobei die Stabilisierungsfläche (20) die Zielfläche (14) an mindestens zwei Punkten berührt, mindestens 1-5 cm von einem Mittelpunkt einer distalen Öffnung des Kanals und auf mindestens zwei nicht-parallelen linearen Achsen.

4. Positioniervorrichtung nach Anspruch 1, wobei das Gehäuse (16) ein oder mehr Vorsprünge (23) angrenzend an den Kanal (18) umfasst, wobei die ein oder mehr Vorsprünge (23) dazu ausgebildet sind, die Injektionsvorrichtung (12) in dem Gehäuse (16) zu halten, wenn die Injektionsvorrichtung (12) in dem Kanal (18) aufgenommen ist.

5. Positioniervorrichtung nach Anspruch 1, ferner umfassend einen mit dem Gehäuse verbundenen Sensor (79), wobei der Sensor dazu ausgebildet ist, einen Zustand des Gehäuses und/oder der Injektionsvorrichtung anhand eines durch den Sensor erfassten Zustands zu erfassen.

6. Positioniervorrichtung nach Anspruch 5, wobei die Positioniervorrichtung mit einer Informationen sendenden, erfassenden und/oder empfangenden Komponente verbunden ist, die dazu ausgebildet ist, Informationen über die Injektionsvorrichtung und/oder das Gehäuse in Bezug auf einen Zustand der Injektionsvorrichtung und/oder des Gehäuses zu empfangen oder zu senden, und optional ferner mindestens eines von einer Signalausgabekomponente, einem Mikroprozessor, einer Speichermediumkomponente und einer Stromquelle umfasst.

7. Positioniervorrichtung nach Anspruch 1, wobei die Positioniervorrichtung ferner entweder eine Komponente zur eindeutigen Identifikation umfasst, wobei die Komponente zur eindeutigen Identifikation Informationen über ein Medikament umfasst, die dazu ausgebildet sind, von einem mit der Injektionsvorrichtung verbundenen Lesegerät zur eindeutigen Identifikation gelesen zu werden, oder ein Lesegerät zur eindeutigen Identifikation, das dazu ausgebildet ist, Informationen über eine mit der Injektionsvorrichtung verbundene Komponente zur eindeutigen Identifikation zu lesen.

8. Positioniervorrichtung nach Anspruch 6, wobei die Positioniervorrichtung dazu ausgebildet ist, Information von einer Datenbank, dem Internet und/oder einem anderen Gerät herunterzuladen oder zu empfangen, wobei die Information Informationen über ein Medikament, Informationen über eine Injektionsvorrichtung einschließlich Anweisungen zur Verwendung, Informationen über eine Kontraindikation von Medikamenten, Informationen über einen Benutzer, Informationen zur Medikamentendosierung, Informationen zur Lagerung, Informationen zu dem verschreibenden Arzt, Informationen zur Apotheke, Herstellerinformationen, Warnhinweise, Rückrufinformationen, Umgebungsdaten, geographische Informationen, Zeitzoneninformationen, MET- bzw. meteorologische Daten, Aufbewahrungsinformationen, Lieferketteninformationen, Versanddaten und/oder Temperaturdaten umfasst.

9. Positioniervorrichtung nach Anspruch 6, wobei die Signalausgabekomponente einen Lautsprecher, ein Display, eine Lampe, eine Vibrationskomponente, eine Geruch emittierende Komponente oder eine die Temperatur ändernde Komponente oder eine Kombination davon umfasst.

10. Positioniervorrichtung nach Anspruch 6, wobei die Positioniervorrichtung Anweisungen zur Verwendung der Positioniervorrichtung und/oder der Injektionsvorrichtung liefern kann, und wobei der Mikroprozessor dazu ausgebildet ist, die Bereitstellung der Anweisungen zur Verwendung der Injektionsvorrichtung und/oder der Positioniervorrichtung für einen Benutzer zu steuern und/oder dem Benutzer eine Anweisung anhand der von der Informationserfassungs- und/oder -empfangskomponente der Positioniervorrichtung erfassten und/oder empfangenen Informationen zu liefern.

11. Positioniervorrichtung nach Anspruch 1, wobei die Führungsschiene (142) relativ zu dem Gehäuse (16) verschieblich ist.

12. Verfahren zum Positionieren einer Injektionsvorrichtung (12) an einem Zielpunkt (t) einer Zielfläche (14) entlang eines ersten Winkels (θ) relativ zu der Zielfläche (14) mit einer repräsentativen Ebene (x), wobei das Verfahren die folgenden Schritte umfasst:
Positionieren einer Positioniervorrichtung mit einem Gehäuse (16) mit einer Führung und einer mit dem Gehäuse (16) verbundenen Führungsschiene (142), wobei die Positioniervorrichtung dazu ausgebildet ist, eine Injektionsvorrichtung (12) aufzunehmen, wobei die Führung einen ersten Winkel zwischen einer Längsachse der Führung und der repräsentativen Ebene der Zielfläche (14) bildet, um eine Injektionsvorrichtung (12) in dem ersten Winkel (θ) zu der Zielfläche (14) zu führen, wobei die Führungsschiene (142) dazu ausgebildet ist, wenigstens einen Abschnitt der Injektionsvorrichtung (12) aufzunehmen und in einer ersten Richtung zu der Zielfläche (14) hin und in einer zweiten Richtung von der Zielfläche (14) weg bewegbar ist,
und wobei das Gehäuse (16) eine Stabilisierungsfläche (20) umfasst, um eine Injektion an der Zielfläche (14) gemäß dem ersten Winkel (θ) zu stabilisieren, sodass die Stabilisierungsfläche (20) des Gehäuses (16) an die Zielfläche (14) stößt, und ein distaler Abschnitt der Führung auf den Zielpunkt (t) der Zielfläche (14) ausgerichtet ist, wobei die Positioniervorrichtung ferner eine Plattform (25) umfasst, die sich von der Stabilisierungsfläche (20) erstreckt, wobei die Plattform (25) einen ersten Abschnitt und einen zweiten Abschnitt umfasst, wobei der erste und der zweite Abschnitt auf beiden Seiten des Gehäuses (16) angeordnet sind, und wobei die Plattform (25) ein biegsames Material umfasst, sodass der erste und der zweite Abschnitt der Plattform (25) wenigstens einen Abschnitt der Zielfläche (14) zwischen dem ersten und dem zweiten Abschnitt der Plattform aufnehmen können, um den Abschnitt der Zielfläche zu verschieben, um die Injektionsvorrichtung (12) aufzunehmen;
Aufnehmen eines Abschnitts der Zielfläche (14) zwischen dem ersten und dem zweiten Abschnitt der Plattform (25);
Einbringen der Injektionsvorrichtung (12) in das Gehäuse (16), sodass sie mit der Führung in dem ersten Winkel (θ) fluchtet; und
Schieben der Injektionsvorrichtung (12) zu dem Zielpunkt (t).

## Revendications

1. Appareil de positionnement pour guider un dispositif d'injection (12) par rapport à une surface cible (14) ayant un plan représentatif, l'appareil comprenant :
un boîtier (16) définissant un canal (18) configuré pour recevoir un dispositif d'injection (12), dans lequel un premier angle (θ) est formé entre un axe longitudinal (y) du canal (18) et le plan représentatif (x) de la surface cible (14) de manière à guider le dispositif d'injection (12) à travers le canal (18) jusqu'à la surface cible (14) au premier angle (θ), et dans lequel ledit boîtier (16) comporte une surface de stabilisation (20) configurée pour venir en butée contre la surface cible (14) afin de stabiliser une injection audit premier angle (θ),
dans lequel le dispositif d'injection (12) comporte un dispositif d'administration de médicament, et dans lequel le canal (18) est configuré pour guider le dispositif d'administration de médicament afin d'administrer une injection à un point cible (t) sur la surface cible (14), de telle sorte qu'un axe longitudinal du dispositif d'administration de médicament est déterminé par l'axe longitudinal du canal (18), et le dispositif d'administration de médicament est en contact avec la surface cible (14) au premier angle (θ), et comprenant en outre une ouverture (22) formée dans la surface de stabilisation (20) du boîtier (16) adjacente à la surface cible (14), dans lequel lorsque le dispositif d'injection (12) est reçu à l'intérieur du canal (18), une partie d'une extrémité distale du dispositif d'injection (12) est alignée sur l'ouverture (22) dans le boîtier (16), de telle sorte qu'un dispositif d'injection (12) comprenant une aiguille (13) à son extrémité distale peut sortir du boîtier (16), dans lequel l'aiguille (13) traverse le boîtier (16) à travers l'ouverture (22),
l'appareil de positionnement comprenant en outre une plate-forme (25) s'étendant à partir de la surface de stabilisation (20), la plate-forme (25) comprenant un axe longitudinal transversal à l'axe longitudinal du canal, dans lequel la plate-forme (25) est configurée pour venir en butée contre la surface cible (14), dans lequel la plate-forme (25) comporte une première partie et une seconde partie, lesdites première et seconde parties sont disposées de part et d'autre du boîtier (16), et ladite plate-forme (25) comporte un matériau souple de telle sorte qu'un utilisateur peut saisir les première et seconde parties de la plate-forme (25) pour recevoir au moins une partie de la surface cible (14) entre les première et seconde parties de la plate-forme afin de déplacer la partie de la surface cible (14) pour recevoir le dispositif d'injection (12),
dans lequel l'appareil de positionnement comporte en outre une glissière de guidage (142) associée au boîtier (16), ladite glissière de guidage (142) étant configurée pour recevoir au moins une partie du dispositif d'injection (12), dans lequel la glissière de guidage (142) guide le dispositif d'injection (12) le long de l'axe longitudinal (y) du canal (18), dans lequel la glissière de guidage (142) est mobile à l'intérieur du canal (18), par rapport au boîtier (16), de telle sorte que lorsque le dispositif d'injection (12) est reçu à l'intérieur de la glissière de guidage (142), la glissière de guidage (142) est mobile dans une première direction se rapprochant de la surface cible (14) et dans une seconde direction s'éloignant de la surface cible (14).

2. Appareil de positionnement selon la revendication 1, dans lequel le premier angle (θ) comporte l'une des valeurs suivantes :
- un angle d'environ 90 degrés,
- un angle entre environ 10 et 15 degrés ou,
- un angle entre environ 40 et 50 degrés.

3. Appareil de positionnement selon la revendication 1, dans lequel le canal (18) a une extrémité proximale (18a) et une extrémité distale (18b), et dans lequel la surface de stabilisation (20) est en contact avec la surface cible (14) en au moins deux points, à au moins 1-5 cm d'un point central d'une ouverture distale du canal et selon au moins deux axes linéaires non parallèles.

4. Appareil de positionnement selon la revendication 1, dans lequel le boîtier (16) comporte une ou plusieurs protubérances (23) adjacentes au canal (18), ladite ou lesdites protubérances (23) étant configurées pour retenir le dispositif d'injection (12) à l'intérieur du boîtier (16) lorsque le dispositif d'injection (12) est reçu à l'intérieur du canal (18).

5. Appareil de positionnement selon la revendication 1, comprenant en outre un capteur (79) associé au boîtier, ledit capteur étant configuré pour détecter un état du boîtier et/ou du dispositif d'injection sur la base d'un état détecté par le capteur.

6. Appareil de positionnement selon la revendication 5, dans lequel l'appareil de positionnement est associé à un composant d'envoi, de détection et/ou de réception d'informations configuré pour recevoir des informations ou envoyer des informations sur le dispositif d'injection et/ou le boîtier liées à un état du dispositif d'injection et/ou du boîtier, et comporte en outre facultativement au moins un élément parmi : un composant de sortie de signal, un microprocesseur, un composant de support de stockage et une source d'alimentation.

7. Appareil de positionnement selon la revendication 1, dans lequel l'appareil de positionnement comporte en outre un composant d'identification unique, ledit composant d'identification unique comprenant des informations sur un médicament, configurées pour être lues par un lecteur d'identification unique associé au dispositif d'injection, ou un lecteur d'identification unique configuré pour lire des informations sur un composant d'identification unique associé au dispositif d'injection.

8. Appareil de positionnement selon la revendication 6, dans lequel l'appareil de positionnement est configuré pour télécharger ou recevoir des informations provenant d'une base de données, d'Internet, et/ou d'un autre dispositif, lesdites informations comprenant des informations sur un médicament, des informations sur un dispositif d'injection incluant des instructions d'utilisation, des informations sur une contre-indication de médicaments, des informations sur un utilisateur, des informations sur un dosage de médicament, des informations de stockage, des informations sur un médecin prescripteur, des informations sur une pharmacie, des informations sur un fabricant, des informations d'avertissement, des informations de rappel, des données environnementales, des informations géographiques, des informations de fuseau horaire, des données météorologiques (MET), des informations de stockage, des informations de chaîne logistique, des données de transit, et/ou des données de température.

9. Appareil de positionnement selon la revendication 6, dans lequel le composant de sortie de signal comporte un haut-parleur, un afficheur, un voyant, un composant de vibration, un composant émettant des odeurs, ou un composant faisant varier la température, ou une combinaison de ceux-ci.

10. Appareil de positionnement selon la revendication 6, dans lequel l'appareil de positionnement peut fournir des instructions pour utiliser l'appareil de positionnement et/ou le dispositif d'injection, et
dans lequel le microprocesseur est configuré pour commander une fourniture des instructions d'utilisation du dispositif d'injection et/ou de l'appareil de positionnement à un utilisateur et/ou pour fournir une instruction à l'utilisateur basée sur des informations détectées et/ou reçues par le composant de détection et/ou de réception d'informations de l'appareil de positionnement.

11. Appareil de positionnement selon la revendication 1, dans lequel la glissière de guidage (142) peut glisser par rapport au boîtier (16).

12. Procédé pour positionner un dispositif d'injection (12) à un point cible (t) d'une surface cible (14) le long d'un premier angle (θ) par rapport à la surface cible (14) ayant un plan représentatif (x), ledit procédé comprenant :
positionner un appareil de positionnement comprenant un boîtier (16) ayant un guide et une glissière de guidage (142) associés au boîtier (16), l'appareil de positionnement étant configuré pour recevoir un dispositif d'injection (12), dans lequel le guide forme un premier angle entre un axe longitudinal du guide et le plan représentatif de la surface cible (14) de manière à guider un dispositif d'injection (12) jusqu'à la surface cible (14) au premier angle (θ), dans lequel la glissière de guidage (142) est configurée pour recevoir au moins une partie du dispositif d'injection (12) et est mobile dans une première direction se rapprochant de la surface cible (14) et dans une seconde direction s'éloignant de la surface cible (14), et
dans lequel ledit boîtier (16) comporte une surface de stabilisation (20) pour stabiliser une injection sur la surface cible (14) selon ledit premier angle (θ), de telle sorte que la surface de stabilisation (20) du boîtier (16) vient en butée contre la surface cible (14), et une partie distale du guide est en alignement avec le point cible (t) de la surface cible (14), l'appareil de positionnement comprenant en outre une plate-forme (25) s'étendant à partir de la surface de stabilisation (20), la plate-forme (25) comprenant une première partie et une seconde partie, lesdites première et seconde parties sont disposées de part et d'autre du boîtier (16), et ladite plate-forme (25) comporte un matériau souple tel que les première et seconde parties de la plate-forme (25) peuvent recevoir au moins une partie de la surface cible (14) entre les première et seconde parties de la plate-forme afin de déplacer la partie de la surface cible pour recevoir le dispositif d'injection (12),
recevoir une partie de la surface cible (14) entre les première et seconde parties de la plate-forme (25),
placer le dispositif d'injection (12) dans le boîtier (16) en alignement avec le guide au premier angle (θ), et
faire glisser le dispositif d'injection (12) jusqu'au point cible (t).
